# EUROPEAN PATENT APPLICATION

(11) **EP 4 821 918 A1**
(43) Date of publication of application: **14.10.2026**
(21) Application number: 24899968.2
(22) Date of filing: 06.12.2024
(51) Int. Cl.: C07D 209/88, A61K 31/404, A61P 21/00

(54) **CO-CRYSTAL FORM OF RNA M6A MODULATOR AND PREPARATION METHOD THEREFOR AND USE THEREOF**

(30) Priority: 08.12.2023 CN 202311684501
(71) Applicant: Suzhou Merna Therapeutics Co. Ltd, Suzhou, Jiangsu 215000 (CN)
(72) Inventor: PENG, Cheng, Suzhou, Jiangsu 215000 (CN); WANG, Jingfang, Suzhou, Jiangsu 215000 (CN); SONG, Zhidong, Suzhou, Jiangsu 215000 (CN); WANG, Ying, Suzhou, Jiangsu 215000 (CN); TONG, Siyu, Suzhou, Jiangsu 215000 (CN)
(74) Representative: Ter Meer und Partner mbB
(86) International application number: PCT/CN2024/137463
(87) International publication number: WO 2025/119335

(57) **Abstract**

The present disclosure provides a co-crystal form of an RNA m6A regulator of formula (I), preparation method therefor and use thereof. The co-crystal form of the RNA m6A regulator according to the present disclosure has the beneficial effects of low hygroscopicity and good stability. The method for preparing the co-crystal form of the RNA m6A regulator has a simple process, an easily controllable crystallization process, and good reproducibility.

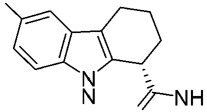

## Description

### CROSS-REFERENCE TO RELATED APPLICATION

The present disclosure claims priority to Chinese patent application No. 202311684501.1, filed with the China National Intellectual Property Administration on December 8, 2023, entitled "CO-CRYSTAL FORM OF RNA m6A REGULATOR AND PREPARATION METHOD THEREFOR AND USE THEREOF", the entire contents of which are incorporated herein by reference.

### TECHNICAL FIELD

The present disclosure belongs to the technical field of medicinal chemistry, and particularly relates to a co-crystal form of an RNA m6A regulator and preparation method therefor and use thereof.

### BACKGROUND

A hand-foot syndrome (HFS) and a hand-foot skin reaction (HFSR) are erythematous skin lesions of palms and soles, which are mainly caused by cytotoxic chemotherapeutic drugs and tumor-targeted drugs, and in severe cases, may lead to loss of self-care ability in patients. Main pathological characteristics of the hand-foot syndrome and the hand-foot skin reaction include vacuolar degeneration of basal keratinocytes, infiltration of perivascular lymphocytes in the skin, apoptosis of keratinocytes, and hydroderma; and under a microscope, inflammatory changes, vasodilation, edema, and leukocyte infiltration can be observed.

Drugs that can cause the hand-foot syndrome include chemotherapeutic drugs such as capecitabine, liposomal doxorubicin, cytarabine, docetaxel, vinorelbine, continuously infused doxorubicin, and gemcitabine; and drugs that can cause the hand-foot skin reaction include targeted drugs such as sunitinib (Sutent), sorafenib (Nexavar), imatinib (Glivec), and erlotinib (Tarceva). HFS is classified by the World Health Organization (WHO) into 4 grades: Grade 1, dysesthesia, paresthesia, or tingling sensation in hands and feet; Grade 2, discomfort, painless swelling, or erythema when holding objects or walking; Grade 3, painful erythema, edema of palms and soles, periungual erythema, and swelling; and Grade 4, desquamation, ulceration, blistering, and severe pain. Currently, severe symptoms of the hand-foot syndrome and the hand-foot skin reaction occurring clinically are often only alleviated by superficial skin care or even require discontinuation of medication. Existing treatments only alleviate the symptoms but not the underlying cause, thereby severely limiting the use of first-line chemotherapeutic drugs for tumors. Therefore, there are significant unmet medical demands. It is required to develop a drug with good efficacy, few side effects, and a low cost for preventing and treating the hand-foot syndrome and the hand-foot skin reaction to meet increasing global medical demands.

m6A methylation modification is a most prevalent RNA modification in mammals, which can regulate multiple signaling pathways and cellular processes (such as growth, development, and diseases), thus playing critical biological actions (Frye M., et al. Science 2018, 361, 2073-2092; Yang C., et al. Cell Death & Disease 2020, 11, 960; Meyer K.D. & Jaffrey S.R. Nature Review Molecular Cell Biology 2014, 15, 313-326). The m6A methylation modification of mRNA, miRNA, circRNA, and lncRNA is a dynamically reversible process, in which methyltransferases (such as METTL3, METTL14, and METTL16) bind methyl to RNA, while demethylases (FTO and ALKBH5) can remove the methyl group from RNA, thus establishing the regulatory basis of m6A modification. m6A affects processes, such as processing, translation, and degradation, of RNA by recruiting specific binding proteins (such as YTHDF1, YTHDF2, YTHDC1, and IGF2BP), thereby resulting in changes in downstream protein functions and cellular biological behaviors (Hsu P.J., et al. Journal of Biological Chemistry 2019, 294, 19889-19895; Yao Y., et al. FASEB Journal 2019, 33, 7529-7544).

Currently, there are few known direct associations between RNA m6A and skin-related diseases, and most of m6A-related methyltransferases or demethylases play roles in skin diseases (especially skin tumors). For example, the mRNA expression level of METTL3 and ALKBH5 in tumor tissues of patients with acral melanoma are significantly higher than those in adjacent non-tumor tissues, and the mRNA expression level of METTL3 in patients with advanced acral melanoma is significantly higher than that in early-stage patients (Le Zhanghui, Preliminary Study on the Pathogenesis of LncRNA and RNA m6A Methylation in Acral Melanoma, Dissertation, 2019). In the aspect of diagnosis of melanoma, m6A-specific binding proteins YTHDF1 and HNRNPA2B1 can serve as novel biomarkers (Li T. D., et al. Cancer Cell 2020, 20, 239). In the aspect of keratinocytes, it has only been reported currently that long-term and low-level arsenic exposure can inhibit selective autophagy caused by the m6A demethylases, thereby inducing the occurrence of skin tumors (Cui Y. H., et al. Nature Communications 2021, 12, 2183). In summary, currently, there are no methods for preventing and treating skin diseases by regulating RNA m6A methylation.

In view of the above, the present disclosure is proposed hereby.

### SUMMARY

### Problems to be solved

Based on unpredictable existence forms and quantities of polymorphic compounds, the present disclosure provides a co-crystal form of an RNA m6A regulator of formula (I), preparation method therefor and use thereof.

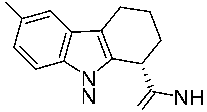

### Technical solutions

The present disclosure provides a pharmaceutical co-crystal formed by a compound of formula (I) and a co-crystal former, where the co-crystal former is selected from nicotinamide, isonicotinamide, L-proline, and glycolic acid,

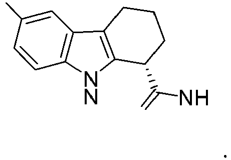

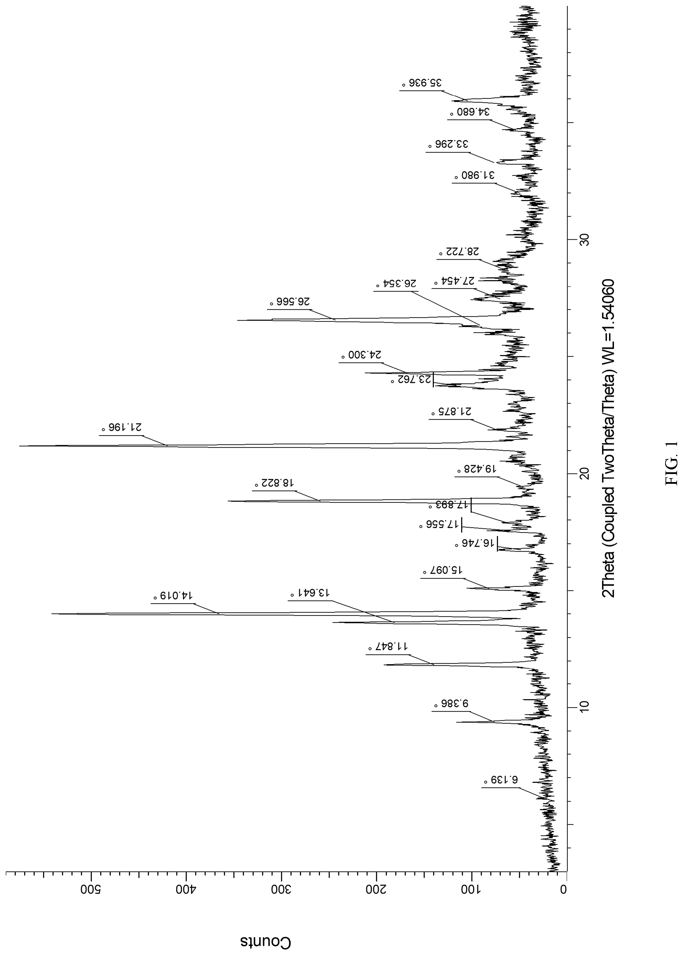

The present disclosure provides a pharmaceutical co-crystal formed by a compound of formula (I) and isonicotinamide, 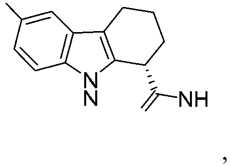 where an X-ray powder diffraction pattern expressed in terms of diffraction angle 2θ has characteristic peaks at 13.641, 14.019, 18.822, 21.196 and 26.566; preferably has characteristic peaks at 9.386, 11.847, 13.641, 14.019, 15.097, 18.822, 21.196, 23.762, 24.300, 26.566 and 35.936; preferably has characteristic peaks at 6.139, 9.386, 11.847, 13.641, 14.019, 15.097, 16.746, 17.556, 17.893, 18.822, 21.196, 23.762, 24.300, 26.354, 26.566, 33.296 and 35.936; and most preferably, the X-ray powder diffraction pattern expressed in terms of diffraction angle 2θ is as shown in FIG. 1.

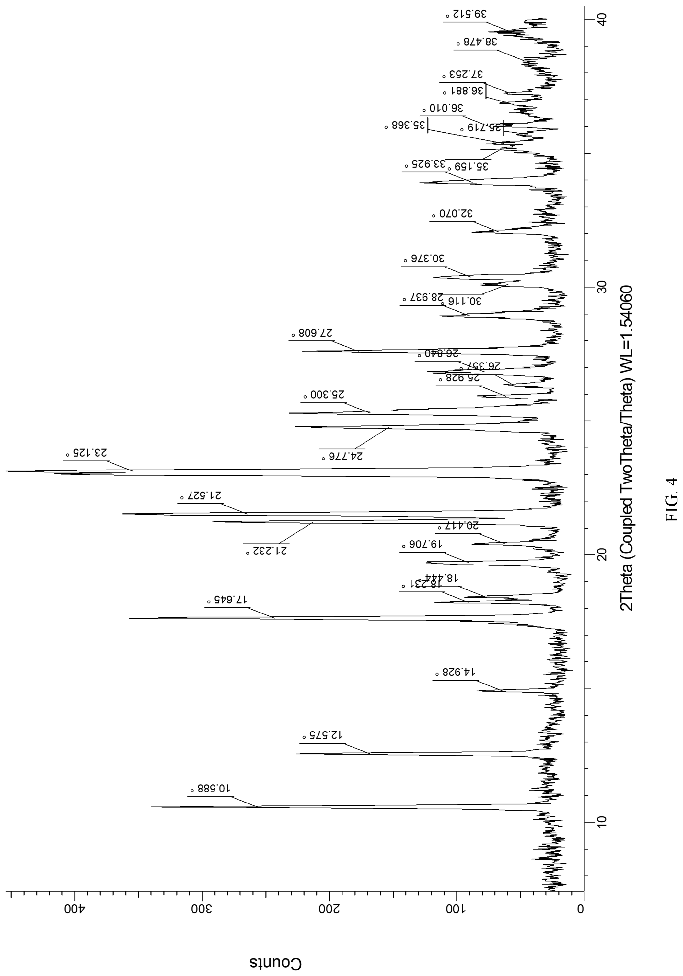

The present disclosure provides a pharmaceutical co-crystal formed by a compound of formula (I) and glycolic acid, 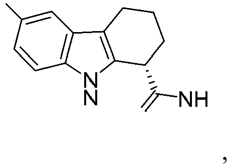 where an X-ray powder diffraction pattern expressed in terms of diffraction angle 2θ has characteristic peaks at 10.588, 17.645, 21.232, 21.527 and 23.125; preferably has characteristic peaks at 10.588, 12.575, 17.645, 18.231, 19.706, 21.232, 21.527, 23.125, 24.776, 25.300 and 27.608; preferably has characteristic peaks at 10.588, 12.575, 14.928, 17.645, 18.231, 18.444, 19.706, 21.232, 21.527, 23.125, 24.776, 25.300, 27.608, 28.937, 30.376 and 33.925; preferably has characteristic peaks at 10.588, 12.575, 14.928, 17.645, 18.231, 18.444, 19.706, 20.417, 21.232, 21.527, 23.125, 24.776, 25.300, 26.840, 27.608, 28.937, 30.116, 30.376, 32.070, 33.925, 35.159, 35.368 and 36.010; and most preferably, the X-ray powder diffraction pattern expressed in terms of diffraction angle 2θ is as shown in FIG. 4.

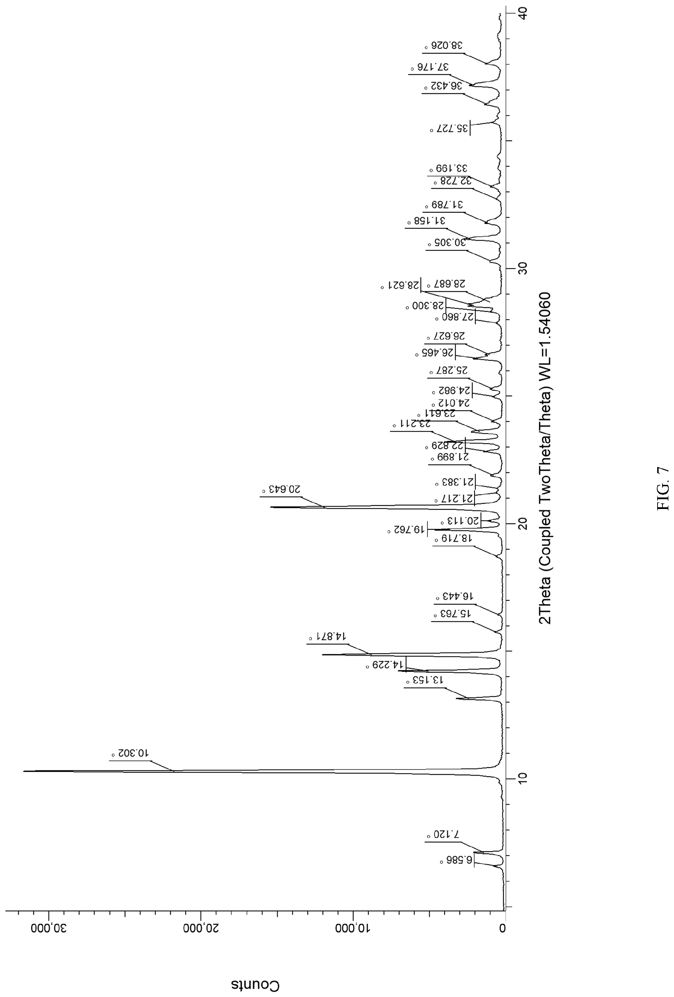

The present disclosure provides a pharmaceutical co-crystal formed by a compound of formula (I) and L-proline, 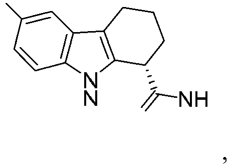 where an X-ray powder diffraction pattern expressed in terms of diffraction angle 2θ has characteristic peaks at 10.302, 14.229, 14.871, 19.762 and 20.643; preferably has characteristic peaks at 7.120, 10.302, 13.153, 14.229, 14.871, 19.762, 20.643, 23.211, 28.621 and 31.158; preferably has characteristic peaks at 6.585, 7.120, 10.302, 13.153, 14.229, 14.871, 19.762, 20.113, 20.643, 22.829, 23.211, 23.611, 26.465, 28.621, 31.158 and 37.176; preferably has characteristic peaks at 6.585, 7.120, 10.302, 13.153, 14.229, 14.871, 15.763, 16.443, 18.719, 19.762, 20.113, 20.643, 21.217, 21.383, 21.899, 22.829, 23.211, 23.611, 26.465, 28.621, 31.158 and 37.176; and most preferably, the X-ray powder diffraction pattern expressed in terms of diffraction angle 2θ is as shown in FIG. 7.

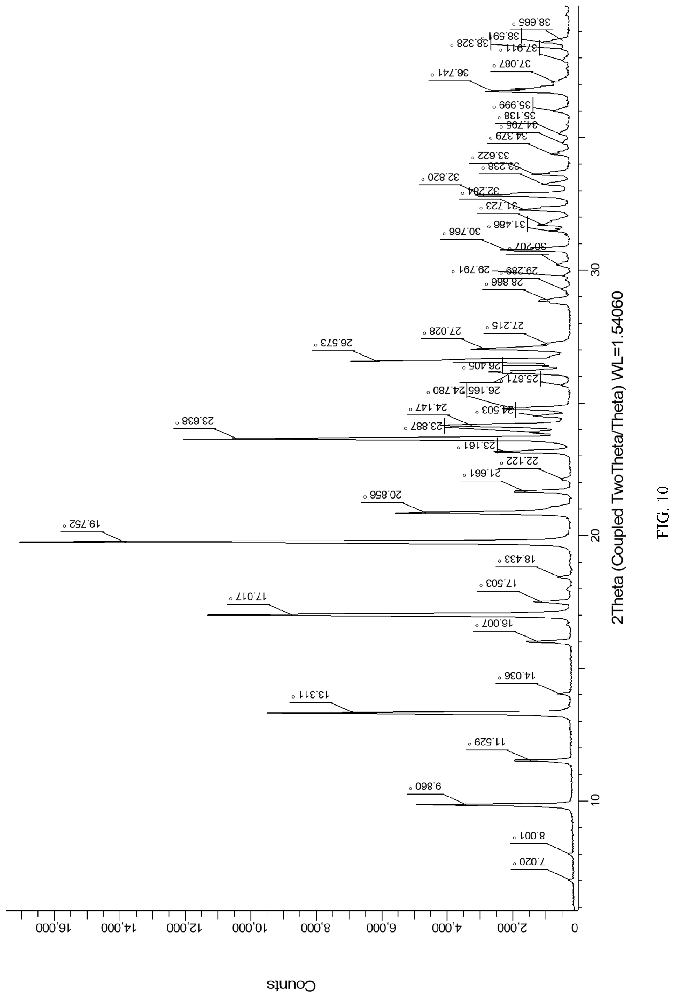

The present disclosure provides a pharmaceutical co-crystal formed by a compound of formula (I) and nicotinamide, 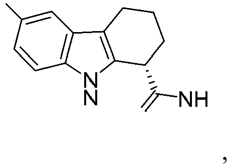 where an X-ray powder diffraction pattern expressed in terms of diffraction angle 2θ has characteristic peaks at 13.311, 17.017, 19.752, 23.638 and 26.573; preferably has characteristic peaks at 9.860, 13.311, 17.017, 19.752, 20.856, 23.638, 24.147, 26.573, 27.028 and 32.820; preferably has characteristic peaks at 9.860, 13.311, 17.017, 19.752, 20.856, 23.161, 23.638, 24.147, 24.780, 26.165, 26.573, 27.028, 30.766, 32.820 and 36.741; preferably has characteristic peaks at 9.860, 11.529, 13.311, 14.036, 16.007, 17.017, 17.503, 19.752, 20.856, 21.661, 23.161, 23.638, 24.147, 24.780, 26.165, 26.573, 27.028, 30.766, 32.284, 32.820 and 36.741; and most preferably, the X-ray powder diffraction pattern expressed in terms of diffraction angle 2θ is as shown in FIG. 10.

### Technical Effects

The pharmaceutical co-crystal formed by the compound of formula (I) and the isonicotinamide, the pharmaceutical co-crystal formed by the compound of formula (I) and the glycolic acid, the pharmaceutical co-crystal formed by the compound of formula (I) and the L-proline, and the pharmaceutical co-crystal formed by the compound of formula (I) and the nicotinamide provided by the present disclosure have advantages in aspects such as physicochemical properties, preparation processability and bioavailability, and for example, have advantages in at least one of aspects such as melting point, solubility, hygroscopicity, purification performance, stability, adhesiveness, compressibility, flowability, *in vivo* and *in vitro* dissolution, and biological effectiveness. The pharmaceutical co-crystal formed by the compound of formula (I) and the co-crystal former in the present disclosure has good physical and chemical self-stability, a high crystal form yield when prepared from the same starting materials, and obvious advantages in aspects such as solubility, hygroscopicity, stability, mechanical stability, flowability, compressibility and adhesiveness, thus providing a new and better option for the development of RNA m6A regulator drugs and having great significance.

### BRIEF DESCRIPTION OF THE DRAWINGS

FIG. 1 is an X-ray powder diffraction (XRPD) pattern of the pharmaceutical co-crystal formed by the compound of formula (I) and the isonicotinamide.

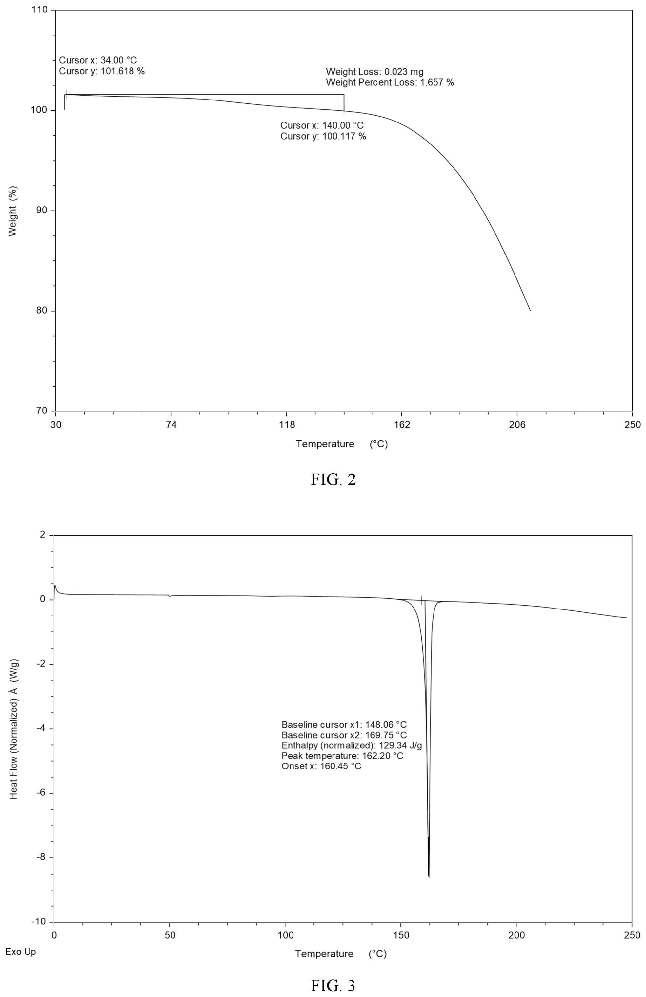

FIG. 2 is a thermogravimetric analysis (TGA) spectrogram of the pharmaceutical co-crystal formed by the compound of formula (I) and the isonicotinamide.
FIG. 3 is a differential scanning calorimetry (DSC) spectrogram of the pharmaceutical co-crystal formed by the compound of formula (I) and the isonicotinamide.
FIG. 4 is an XRPD pattern of the pharmaceutical co-crystal formed by the compound of formula (I) and the glycolic acid.

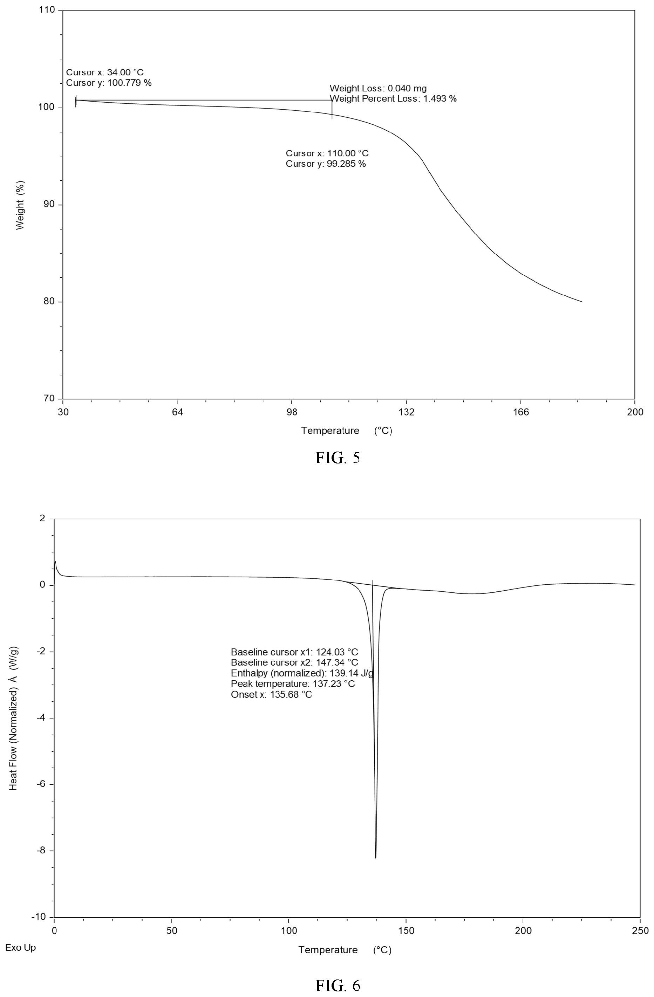

FIG. 5 is a TGA spectrogram of the pharmaceutical co-crystal formed by the compound of formula (I) and the glycolic acid.
FIG. 6 is a DSC spectrogram of the pharmaceutical co-crystal formed by the compound of formula (I) and the glycolic acid.
FIG. 7 is an XRPD pattern of the pharmaceutical co-crystal formed by the compound of formula (I) and the L-proline.

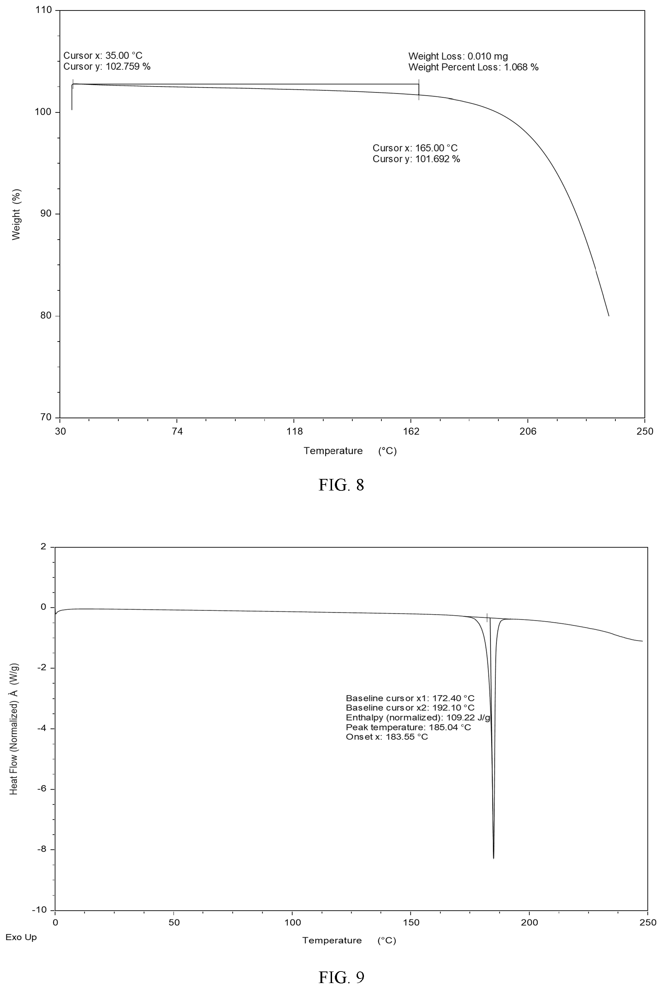

FIG. 8 is a TGA spectrogram of the pharmaceutical co-crystal formed by the compound of formula (I) and the L-proline.
FIG. 9 is a DSC spectrogram of the pharmaceutical co-crystal formed by the compound of formula (I) and the L-proline.
FIG. 10 is an XRPD pattern of the pharmaceutical co-crystal formed by the compound of formula (I) and the nicotinamide.

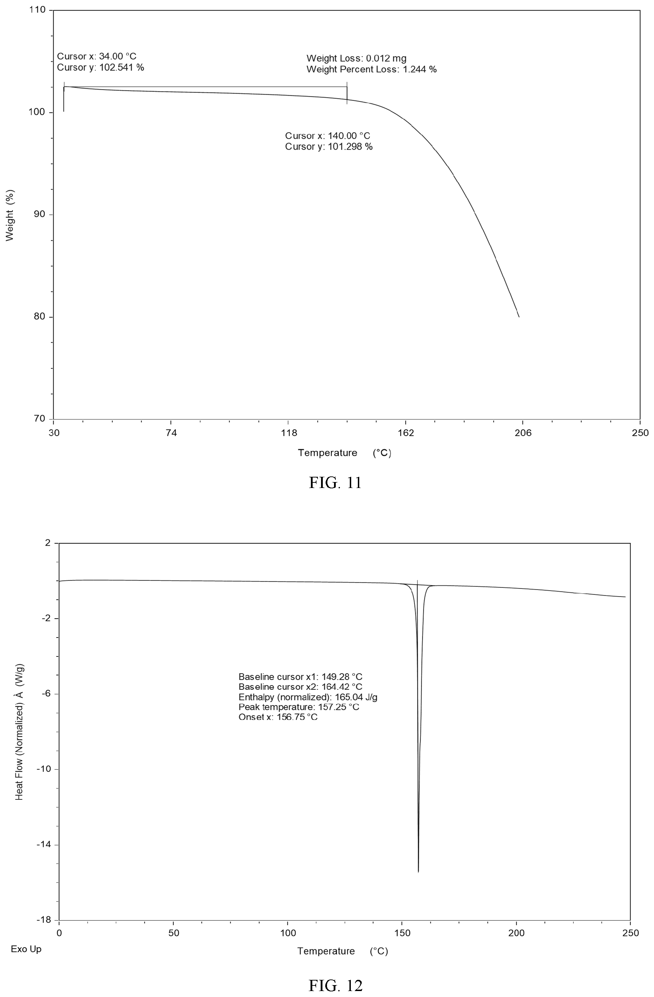

FIG. 11 is a TGA spectrogram of the pharmaceutical co-crystal formed by the compound of formula (I) and the nicotinamide.
FIG. 12 is a DSC spectrogram of the pharmaceutical co-crystal formed by the compound of formula (I) and the nicotinamide.
FIG. 13 describes exemplary results of a cellular m6A level and an NAP1L2 m6A level determined by nanopore sequencing after administration of sorafenib, capecitabine, docetaxel, and osimertinib to HaCaT keratinocytes for 24 hours.
FIG. 14 describes exemplary results of a cellular m6A level and an NAP1L2 m6A level in skin tissues of hind limb toes of mice in Example 3.
FIG. 15 describes exemplary results of an NAP1L2 mRNA expression level determined by reverse transcription-polymerase chain reaction (RT-PCR) after administration of a recombinant human METTL3 protein and a recombinant human FTO protein or transfection with METTL3 siRNA and FTO siRNA to HaCaT keratinocytes for 24 hours.
FIG. 16 describes exemplary results of mRNA and protein expression amounts of matrix metalloproteinases determined by RT-PCR and Western blot after administration of a recombinant human NAP1L2 protein, sorafenib, capecitabine, docetaxel, and osimertinib or transfection with NAP1L2 siRNA to HaCaT keratinocytes for 24 hours.
FIG. 17 describes exemplary results of mRNA and protein expression amounts of keratinocyte differentiation markers KRT1, KRT10, Loricrin, and Involucrin determined by RT-PCR and Western blot after administration of a recombinant human HBEGF protein and an HBEGF antibody or transfection with METTL3 and NAP1L2 siRNA to HaCaT keratinocytes for 24 hours.
FIG. 18 describes exemplary results of a cellular m6A methylation inhibition level determined by liquid chromatography-mass spectrometry (LC-MS) after administration of a compound of formula (I), nicotinamide, and UZH2 to THP-1 cells for 24 hours.
FIG. 19 describes exemplary results of mRNA expression level of keratinocyte differentiation markers KRT1, KRT10, Loricrin, and Involucrin determined by RT-PCR after administration of a recombinant human HBEGF protein and a compound of formula (I) to HaCaT keratinocytes for 24 hours.
FIG. 20 describes exemplary stratum corneum thickness measurement results of paw plantar skin of rats in Example 10.
FIG. 21 describes exemplary histopathological results of epithelial blisters, inflammatory cell infiltration, and skin tissue congestion situations of paw plantar skin of rats after tissue staining in Example 10.
FIG. 22 describes exemplary histopathological scoring results of the epithelial blisters, the inflammatory cell infiltration, and the skin tissue congestion situations of the paw plantar skin of the rats after tissue staining in Example 10.
FIG. 23 describes exemplary results of the skin of hind limb toes of mice in Example 11.
FIG. 24 describes exemplary results of the degree of swelling of hind limb toes of mice in Example 11.
FIG. 25 describes exemplary histopathological results of skin tissues of hind limb toes of mice in Example 11.
FIG. 26 describes exemplary immunohistochemical results of cytokine IL-8 in skin tissues of hind limb toes of mice in capecitabine modeling series groups in Example 11.
FIG. 27 describes exemplary immunohistochemical results of cytokine IL-6 in skin tissues of hind limb toes of mice in docetaxel modeling series groups in Example 11.
FIG. 28 describes exemplary results of the skin of hind limb toes of mice in Example 12.
FIG. 29 describes exemplary results of the degree of swelling of hind limb toes of mice in Example 12.
FIG. 30 describes exemplary histopathological results of skin tissues of hind limb toes of mice in Example 12.
FIG. 31 describes exemplary immunohistochemical results of cytokine IL-1β in skin tissues of hind limb toes of mice in sorafenib modeling series groups in Example 12.
FIG. 32 describes exemplary immunohistochemical results of cytokine IL-1β in skin tissues of hind limb toes of mice in osimertinib modeling series groups in Example 12.

### DETAILED DESCRIPTION OF THE EMBODIMENTS

To make the technical solutions and beneficial effects of the present disclosure more apparent and understandable, the following provides a detailed description by the way of listing specific examples. The drawings are not necessarily drawn to scale, and local features may be enlarged or reduced to more clearly show details of the local features. Unless otherwise defined, technical and scientific terms used herein have the same meanings as those in the technical field to which the present disclosure belongs.

The present disclosure provides a pharmaceutical co-crystal formed by a compound of formula (I) and isonicotinamide, 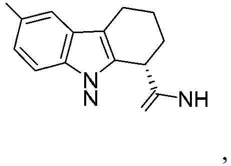 where an X-ray powder diffraction pattern expressed in terms of diffraction angle 2θ has characteristic peaks at 13.641, 14.019, 18.822, 21.196 and 26.566; preferably has characteristic peaks at 9.386, 11.847, 13.641, 14.019, 15.097, 18.822, 21.196, 23.762, 24.300, 26.566 and 35.936; preferably has characteristic peaks at 6.139, 9.386, 11.847, 13.641, 14.019, 15.097, 16.746, 17.556, 17.893, 18.822, 21.196, 23.762, 24.300, 26.354, 26.566, 33.296 and 35.936; and most preferably, the X-ray powder diffraction pattern expressed in terms of diffraction angle 2θ is as shown in FIG. 1.

The present disclosure further provides a method for preparing the pharmaceutical co-crystal formed by the compound of formula (I) and the isonicotinamide, which includes: mixing the compound of formula (I) with the isonicotinamide, adding solvent 1, stirring, and filtering.

In some embodiments, the solvent 1 is selected from alcohol solvent.

In some embodiments, the solvent 1 is selected from C₁₋₄ alcohol.

In some embodiments, the solvent 1 is selected from one or more of methanol, ethanol, and isopropanol.

In some embodiments, the solvent 1 is selected from ethanol.

In some embodiments, the preparation method of the present disclosure further includes steps such as centrifugation, washing, or drying.

The present disclosure further provides a pharmaceutical co-crystal formed by a compound of formula (I) and glycolic acid, 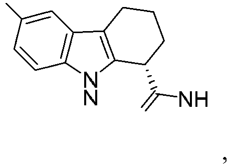 where an X-ray powder diffraction pattern expressed in terms of diffraction angle 2θ has characteristic peaks at 10.588, 17.645, 21.232, 21.527 and 23.125; preferably has characteristic peaks at 10.588, 12.575, 17.645, 18.231, 19.706, 21.232, 21.527, 23.125, 24.776, 25.300 and 27.608; preferably has characteristic peaks at 10.588, 12.575, 14.928, 17.645, 18.231, 18.444, 19.706, 21.232, 21.527, 23.125, 24.776, 25.300, 27.608, 28.937, 30.376 and 33.925; preferably has characteristic peaks at 10.588, 12.575, 14.928, 17.645, 18.231, 18.444, 19.706, 20.417, 21.232, 21.527, 23.125, 24.776, 25.300, 26.840, 27.608, 28.937, 30.116, 30.376, 32.070, 33.925, 35.159, 35.368 and 36.010; and most preferably, the X-ray powder diffraction pattern expressed in terms of diffraction angle 2θ is as shown in FIG. 4.

The present disclosure further provides a method for preparing the pharmaceutical co-crystal formed by the compound of formula (I) and the glycolic acid, which includes: mixing the compound of formula (I) with the glycolic acid, adding solvent 2, stirring, and filtering.

In some embodiments, the solvent 2 is selected from ester solvent.

In some embodiments, the solvent 2 is selected from one or more of ethyl acetate, n-propyl acetate, isopropyl acetate, n-butyl acetate, and isobutyl acetate.

In some embodiments, the solvent 2 is selected from isopropyl acetate.

In some embodiments, the preparation method of the present disclosure further includes steps such as centrifugation, washing, or drying.

The present disclosure further provides a pharmaceutical co-crystal formed by a compound of formula (I) and L-proline, 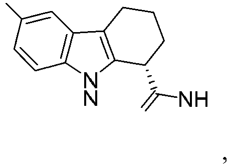 where an X-ray powder diffraction pattern expressed in terms of diffraction angle 2θ has characteristic peaks at 10.302, 14.229, 14.871, 19.762 and 20.643; preferably has characteristic peaks at 7.120, 10.302, 13.153, 14.229, 14.871, 19.762, 20.643, 23.211, 28.621 and 31.158; preferably has characteristic peaks at 6.585, 7.120, 10.302, 13.153, 14.229, 14.871, 19.762, 20.113, 20.643, 22.829, 23.211, 23.611, 26.465, 28.621, 31.158 and 37.176; preferably has characteristic peaks at 6.585, 7.120, 10.302, 13.153, 14.229, 14.871, 15.763, 16.443, 18.719, 19.762, 20.113, 20.643, 21.217, 21.383, 21.899, 22.829, 23.211, 23.611, 26.465, 28.621, 31.158 and 37.176; and most preferably, the X-ray powder diffraction pattern expressed in terms of diffraction angle 2θ is as shown in FIG. 7.

The present disclosure further provides a method for preparing the pharmaceutical co-crystal formed by the compound of formula (I) and the L-proline, which includes: mixing the compound of formula (I) with the L-proline, adding solvent 3, stirring, and filtering.

In some embodiments, the solvent 3 is selected from alcohol solvent.

In some embodiments, the solvent 3 is selected from C₁₋₄ alcohol.

In some embodiments, the solvent 3 is selected from one or more of methanol, ethanol, and isopropanol.

In some embodiments, the solvent 3 is selected from ethanol.

In some embodiments, the preparation method of the present disclosure further includes steps such as centrifugation, washing, or drying.

The present disclosure further provides a pharmaceutical co-crystal formed by a compound of formula (I) and nicotinamide,

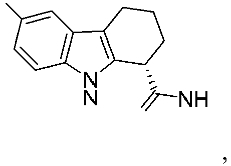

where an X-ray powder diffraction pattern expressed in terms of diffraction angle 2θ has characteristic peaks at 13.311, 17.017, 19.752, 23.638 and 26.573; preferably has characteristic peaks at 9.860, 13.311, 17.017, 19.752, 20.856, 23.638, 24.147, 26.573, 27.028 and 32.820; preferably has characteristic peaks at 9.860, 13.311, 17.017, 19.752, 20.856, 23.161, 23.638, 24.147, 24.780, 26.165, 26.573, 27.028, 30.766, 32.820 and 36.741; preferably has characteristic peaks at 9.860, 11.529, 13.311, 14.036, 16.007, 17.017, 17.503, 19.752, 20.856, 21.661, 23.161, 23.638, 24.147, 24.780, 26.165, 26.573, 27.028, 30.766, 32.284, 32.820 and 36.741; and most preferably, the X-ray powder diffraction pattern expressed in terms of diffraction angle 2θ is as shown in FIG. 10.

The present disclosure further provides a method for preparing the pharmaceutical co-crystal formed by the compound of formula (I) and the nicotinamide, which include: mixing the compound of formula (I) with the nicotinamide, adding solvent 4, stirring, and filtering.

In some embodiments, the solvent 4 is selected from nitrile solvent.

In some embodiments, the solvent 4 is selected from one or more of acetonitrile, trimethylacetonitrile, propionitrile, and valeronitrile.

In some embodiments, the solvent 4 is selected from acetonitrile.

In some embodiments, the preparation method of the present disclosure further includes steps such as centrifugation, washing, or drying.

The present disclosure further provides a pharmaceutical composition prepared from the aforementioned pharmaceutical co-crystal formed by the compound of formula (I) and the co-crystal former, the aforementioned pharmaceutical co-crystal formed by the compound of formula (I) and the isonicotinamide, the aforementioned pharmaceutical co-crystal formed by the compound of formula (I) and the glycolic acid, the aforementioned pharmaceutical co-crystal formed by the compound of formula (I) and the L-proline, or the aforementioned pharmaceutical co-crystal formed by the compound of formula (I) and the nicotinamide.

The present disclosure further provides a pharmaceutical composition, which includes the aforementioned pharmaceutical co-crystal formed by the compound of formula (I) and the co-crystal former, the aforementioned pharmaceutical co-crystal formed by the compound of formula (I) and the isonicotinamide, the aforementioned pharmaceutical co-crystal formed by the compound of formula (I) and the glycolic acid, the aforementioned pharmaceutical co-crystal formed by the compound of formula (I) and the L-proline, or the aforementioned pharmaceutical co-crystal formed by the compound of formula (I) and the nicotinamide, and optionally a pharmaceutically acceptable excipient.

The "pharmaceutically acceptable excipient" refers to a pharmaceutically acceptable material, mixture, or vehicle related to the consistency of a dosage form or the pharmaceutical composition. Suitable pharmaceutically acceptable excipients are different according to selected dosage forms. Furthermore, the pharmaceutically acceptable excipients may be selected according to their specific functions in the composition.

In some embodiments, the pharmaceutically acceptable excipient includes excipients of the following types: a diluent, a filler, a penetration enhancer, a binder, a disintegrant, a lubricant, a glidant, a granulating agent, a coating agent, a wetting agent, a solvent, a cosolvent, a suspending aid, an emulsifier, a flavoring agent, a taste-masking agent, a colorant, an anti-caking agent, a humectant, a chelating agent, a plasticizer, a tackifier, an antioxidant, a preservative, a stabilizer, a surfactant, and a buffer.

In some embodiments, the pharmaceutical composition is a solid preparation.

In some embodiments, the pharmaceutical composition is a film and a coated film.

In some embodiments, the pharmaceutical composition is an ointment.

In some embodiments, the pharmaceutical composition is a plaster.

In some embodiments, the pharmaceutical composition is a cream.

In some embodiments, the solid preparation is a capsule.

In some embodiments, the pharmaceutical composition is a tablet.

In some embodiments, the ointment contains the co-crystal of the compound of formula (I) in an amount of 0-50% by weight.

The present disclosure further provides a method for preparing a pharmaceutical composition, which includes a step of mixing the aforementioned pharmaceutical co-crystal formed by the compound of formula (I) and the co-crystal former, the aforementioned pharmaceutical co-crystal formed by the compound of formula (I) and the isonicotinamide, the aforementioned pharmaceutical co-crystal formed by the compound of formula (I) and the glycolic acid, the aforementioned pharmaceutical co-crystal formed by the compound of formula (I) and the L-proline, or the aforementioned pharmaceutical co-crystal formed by the compound of formula (I) and the nicotinamide with a pharmaceutically acceptable excipient.

The present disclosure further provides use of the aforementioned pharmaceutical co-crystal formed by the compound of formula (I) and the co-crystal former, the aforementioned pharmaceutical co-crystal formed by the compound of formula (I) and the isonicotinamide, the aforementioned pharmaceutical co-crystal formed by the compound of formula (I) and the glycolic acid, the aforementioned pharmaceutical co-crystal formed by the compound of formula (I) and the L-proline, the aforementioned pharmaceutical co-crystal formed by the compound of formula (I) and the nicotinamide, the aforementioned composition, or the composition prepared by the aforementioned method in preparation of a drug for treating and/or preventing a disease related to RNA m6A regulation.

In some embodiments, the disease related to m6A regulation is a disease or pain related to endocrine and metabolic diseases, neurological diseases, tumors, cardiovascular diseases, infections, immune system diseases, urogenital diseases, dermatological and musculoskeletal diseases, respiratory diseases, genetic diseases and malformations, digestive system diseases, stomatognathic diseases, and vascular and lymphatic system diseases.

In some embodiments, the disease related to m6A regulation is a hand-foot syndrome, a hand-foot skin reaction, a cancer, or a dermatological disease.

In the present disclosure, the "2θ or 2θ angle" refer to the diffraction angle, where θ is a Bragg angle, in the unit of ° or degree; and an error margin for each characteristic peak at 2θ is ±0.2 degrees (including cases where numbers with more than 1 decimal place are rounded), which may be -0.20, - 0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, a precipitation manner includes, but is not limited to, stirring, cooling, volatilization, slurrying, and precipitation.

The " slurrying " is a commonly used noun in the field of pharmaceutical preparation, generally referring to mechanical or fluidic processing of a solid pharmaceutical raw material to disperse or suspend a solid drug in a solvent.

In some embodiments, a slurrying time is 5-30 h.

According to the description of hygroscopicity characteristics and the definition of hygroscopic weight gain in the "9103 Guidelines for the Hygroscopicity of Pharmaceuticals" in Part IV of the 2020 Edition of the Chinese Pharmacopoeia,
deliquescence represents absorption of a sufficient amount of water to form a liquid;
high hygroscopicity represents a hygroscopic weight gain not less than 15%;
hygroscopicity represents a hygroscopic weight gain less than 15% but not less than 2%;
slight hygroscopicity represents a hygroscopic weight gain less than 2% but not less than 0.2%; and
no hygroscopicity or almost no hygroscopicity represents a hygroscopic weight gain less than 0.2%.

In the present disclosure, the "differential scanning calorimetry or DSC" refers to measuring a temperature difference and a heat flow difference between a sample and a reference during a heating or constant temperature process of the sample to characterize all physical changes and chemical changes related to a thermal effect and obtain phase transition information of the sample.

In the present disclosure, a drying temperature is generally 25°C-100°C, preferably 40°C-70°C, and the drying may be drying under ambient pressure or drying under reduced pressure.

The methods of the present disclosure are described below through specific examples. It should be understood that these examples are used to illustrate basic principles, main features, and advantages of the present disclosure, and the scope of the present disclosure is not limited to the following examples. Implementation conditions used in the examples may be further adjusted according to specific requirements, and unspecified implementation conditions usually are conditions in conventional experiments.

Explanations of abbreviations used in the present disclosure are as follows:
XRPD: X-ray powder diffraction;
DSC: differential scanning calorimetry;
TGA: thermogravimetric analysis;
DVS: dynamic vapor sorption; and
HPLC: high performance liquid chromatography.
Detection instruments and methods
X-ray powder diffraction (XRPD)

Crystal form analysis of a sample was performed using a Bruker D8 ADVANCE X-ray powder diffractometer. A 2θ scanning angle of the sample was 3°-40°, a scanning step size was 0.02°, and a scanning time per step was 0.12 s/step. An optical tube voltage and a current were 40 kV and 40 mA, respectively. During sample preparation, an appropriate amount of the sample was placed on a sample tray and flattened with a tool such as a glass slide to ensure a smooth and even surface.

### Thermogravimetric analysis (TGA)

TA Instruments TGA Discovery 5500 was used for analysis of a sample. The sample was placed in a tared aluminum tray, automatically weighed by the system, and then heated to a specified temperature at a rate of 10°C/min under the protection of nitrogen.

### Differential scanning calorimetry (DSC)

TA Instruments Discovery 2500 was used for analysis of a sample. 0.5-1.5 mg of the sample was weighed, placed in a sample tray, and then heated to a specified temperature at a rate of 10°C/min under the protection of nitrogen (50 ml/min).

### Dynamic vapor sorption analysis (DVS)

ProUmid SPSx-1µ Advance was used for analysis of a sample. The sample to be tested was approximately in an amount of 5-50 mg. A temperature in a test chamber was controlled at 25±1°C, a relative humidity cycle was 40-95-0-95-40%, a step size was 10%, an equilibrium time was 240 minutes per step, and mass data was recorded every 20 seconds.

### High performance liquid chromatography (HPLC)

An Agilent 1260 infinityII binary pump was used for solubility and stability testing.

### Example 1: Preparation of the compound of formula (I)

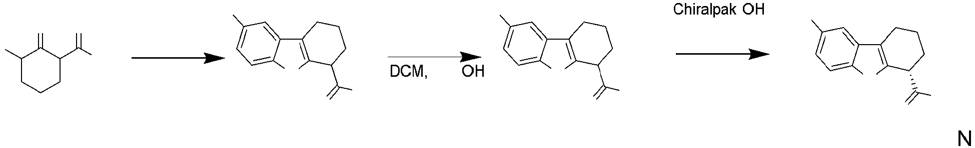

Ethyl 3-bromo-2-oxocyclohexane-1-formate (20 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 N HCl, and washed 1 time with 100 mL of saturated NaHCO₃. The organic layer was dried over anhydrous Na₂SO₄, concentrated under vacuum, and separated and purified by silica gel column chromatography to obtain ethyl 6-chloro-2,3,4,9-tetrahydro-1H-carbazole-1-formate (16 mg). 10 mg of the ethyl 6-chloro-2,3,4,9-tetrahydro-1H-carbazole-1-formate (0.036 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, adjusted to a pH of 2, and then extracted 3 times with 50 mL of dichloromethane. The organic phases were combined, dried, and concentrated, followed by the addition of ammonia water and heating at 60°C for a reaction for 24 hours. The mixture was separated and purified by silica gel column chromatography to obtain 6-chloro-2,3,4,9-tetrahydro-1H-carbazole-1-carboxamide as a white solid (compound 1, 7.6 mg), which was separated by a Chiralpak AD chiral column to obtain (S)-6-chloro-2,3,4,9-tetrahydro-1H-carbazole-carboxamide (the compound of formula (I)). LCMS [M+H]⁺ of the compound of formula (I): 249. ¹H-NMR (400 MHz, DMSO-*d*₆): δ 10.79 (s, 1H), 7.37-7.39 (m, 2H), 7.28 (d, *J* = 8.0 Hz, 1H), 7.08 (s, 1H), 6.98-7.01 (m, 1H), 3.64-3.67 (m, 1H), 2.58-2.61 (m, 2H), 1.66-2.04 (m, 4H).

### Example 2: Changes in an mRNA methylation level of drug-induced keratinocytes

1*10⁸ HaCaT cells were seeded in culture dishes, into which 50 nM drugs (including sorafenib, capecitabine, docetaxel, and osimertinib) were added, respectively. The cells were cultured in an incubator containing 5% CO₂ at 37°C for 24 hours and washed with PBS after culture media were removed. Centrifugation was performed, the supernatant was discarded, and then, 400 µL of an RLT cell lysis buffer (QIAGEN, Cat.#79216) was added. A portion of samples were selected, into which 400 µL of 70% ethanol was added. The mixture was evenly stirred and then transferred to an RNeasy mini column, and RNA was extracted using an RNeasy mini kit (QIAGEN, Cat.#74104). 2 µL of an RNA sample was selected and dissolved in 98 µL of a 10 mM Tris-HCl buffer solution, and the absorbance at 260 nm was measured using a Nanodrop (Thermo Fisher Scientific) to determine the RNA concentration.

40 µg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit^{™} dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

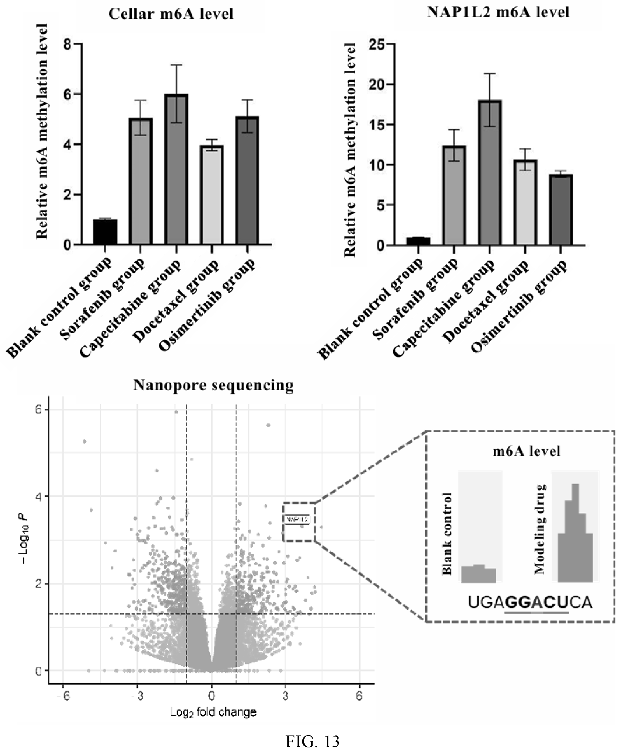

Results are shown in FIG. 13. Compared with a blank control group, the m6A methylation levels of HaCaT cells were 5.05-fold, 6.01-fold, 3.97-fold, 5.13-fold higher than that of the control group after the addition of the sorafenib, the capecitabine, the docetaxel, and the osimertinib, respectively. This indicates that antitumor drugs (including the sorafenib, the capecitabine, the docetaxel, and the osimertinib) induced the abnormal m6A methylation of mRNA of human keratinocytes. It is further found through bioinformatics analysis that the m6A methylation level of NAP1L2 had a significant difference, mainly showing in the m6A methylation of a UGAGGACUCA fragment.

### Example 3: Changes in an mRNA methylation level in drug-induced mouse models of adverse skin reactions

After adaptive feeding for 7 days, male ICR mice (5-6 weeks old, with a body weight of approximately 35 g) were randomly divided into groups, with 6 mice in each group, including a blank control group, a sorafenib group, a capecitabine group, a docetaxel group, and an osimertinib group. The groups were respectively administered corresponding modeling drugs by gavage (sorafenib at 100 mg/kg, capecitabine at 200 mg/kg, docetaxel at 25 mg/kg, and osimertinib at 10 mg/kg) once daily. After continuous administration for 30 days, the mice were sacrificed, and skin tissues of hind limb toes of the mice were collected. A small amount of a tissue sample was selected, placed in a mortar containing liquid nitrogen, and ground into powder. A single-phase lysis buffer was added. After being placed at room temperature for 5 minutes, the sample was centrifuged (12,000 rpm) for 5 minutes. 1 mL of the supernatant was collected, 200 µL of chloroform was added, and the mixture was evenly mixed by shaking, placed at room temperature for 15 minutes, and centrifuged (12,000 rpm, 4°C) for 15 minutes. An aqueous phase on an upper layer was sucked, added into an equal volume of isopropanol, placed at -20°C for 1 hour, and then centrifuged (12,000 rpm, 4°C) to discard the supernatant. 400 µL of 70% ethanol was added to a precipitate, the mixture was evenly stirred and then transferred to an RNeasy mini column, and RNA was extracted using an RNeasy mini kit (QIAGEN, Cat.#74104). 2 µL of an RNA sample was selected and dissolved in 98 µL of a 10 mM Tris-HCl buffer solution, and the absorbance at 260 nm was measured using a Nanodrop (Thermo Fisher Scientific) to determine the RNA concentration.

40 µg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit^{TM} dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

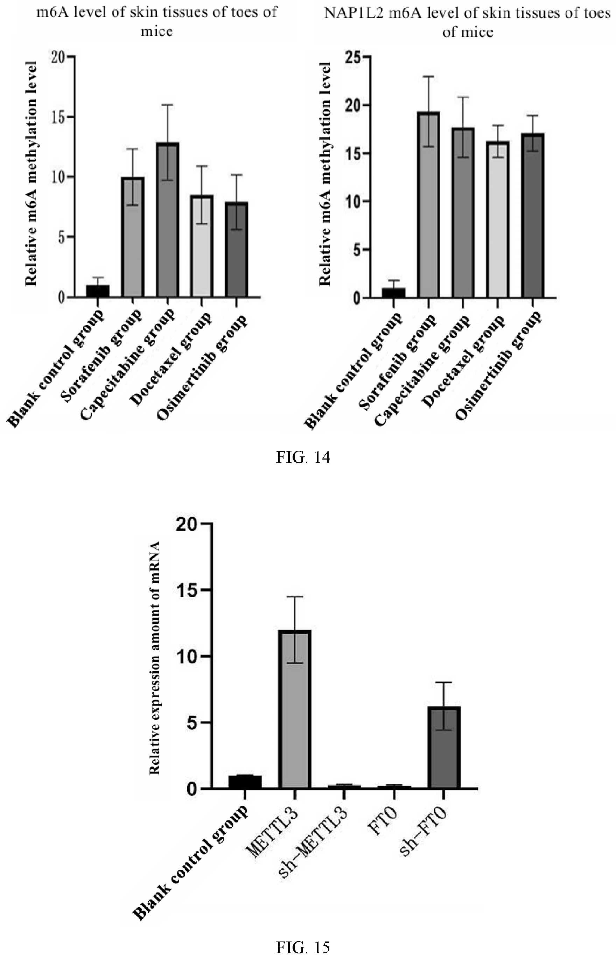

Results are shown in FIG. 14. Compared with a positive control group, the m6A methylation levels of skin tissues of hind limb toes of the mice in the drug modeling groups were significantly increased. The m6A methylation level of NAP1L2 in the drug addition groups had a significant difference from the blank control group.

### Example 4: Regulation of an mRNA expression level of NAP1L2 by m6A

1*10⁸ HaCaT cells were seeded in culture dishes, into which a recombinant protein of m6A methyltransferase METTL3 (Abcam, Cat.#ab271611) and a recombinant protein of m6A demethylase FTO (Abcam, Cat.#ab271525) were added, or METTL3 siRNA (sh-METTL3, GeneChem) and FTO siRNA (sh-FTO, GeneChem) were transfected, respectively. The cells were placed in an incubator containing 5% carbon dioxide for culture at 37°C for 24 hours, washed with PBS, and then collected by high-speed centrifugation. Total RNA was extracted using Trizol reagent (Sigma Aldrich). 1 µg of RNA was reverse-transcribed into cDNA using a cDNA reverse transcription kit (Transgene Biotech, Cat.#AT311-03). 1.25 µL of a primer (Beyotime, Cat.#QH18721S), 10 µL of an iTag Universal SYBR Green supermix (Bio-Rad, Cat.#172-5125), and an appropriate amount of DEPC ultrapure water were added to formulate 20 µL of a reaction solution to perform an RT-PCR reaction. Upon completion of the reaction, the reaction solution was subjected to agarose gel electrophoresis to determine the mRNA expression level of NAP1L2.

Results are shown in FIG. 15. When the m6A methyltransferase METTL3 was added to the HaCaT keratinocytes or the function of the m6A demethylase FTO was inhibited (via sh-FTO), the mRNA expression level of NAP1L2 was significantly increased; and when the m6A demethylase FTO was added to the HaCaT keratinocytes or a function of the m6A methyltransferase METTL3 was inhibited (via sh-METTL3), the mRNA expression level of NAP1L2 was significantly decreased. This indicates that m6A methylation regulated the mRNA expression level of NAP1L2.

### Example 5: Regulation of the expression of matrix metalloproteinases (MMPs) expression in human HaCaT keratinocytes by NAP1L2

1*10⁸ HaCaT cells were seeded in culture dishes, into which a recombinant human NAP1L2 protein (Abcam, Cat.#ab117213), sorafenib, capecitabine, docetaxel, and osimertinib were added, or NAP1L2 siRNA (sh-NAP1L2, GeneChem) was transfected, respectively. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the supernatant, and the expression of matrix metalloproteinases (MMPs) was measured by Western blot.

Total RNA was extracted from the above collected cells using Trizol reagent (Sigma Aldrich). 1 µg of RNA was reverse-transcribed into cDNA using a cDNA reverse transcription kit (Transgene Biotech, Cat.#AT311-03). 1.25 µL of a primer (SinoBiological, Cat.#HP100168&HP100367), 10 µL of an iTag Universal SYBR Green supermix (Bio-Rad, Cat.#172-5125), and an appropriate amount of DEPC ultrapure water were added to formulate 20 µL of a reaction solution to perform an RT-PCR reaction. Upon completion of the reaction, the reaction solution was subjected to agarose gel electrophoresis to determine the mRNA expression level of the matrix metalloproteinases.

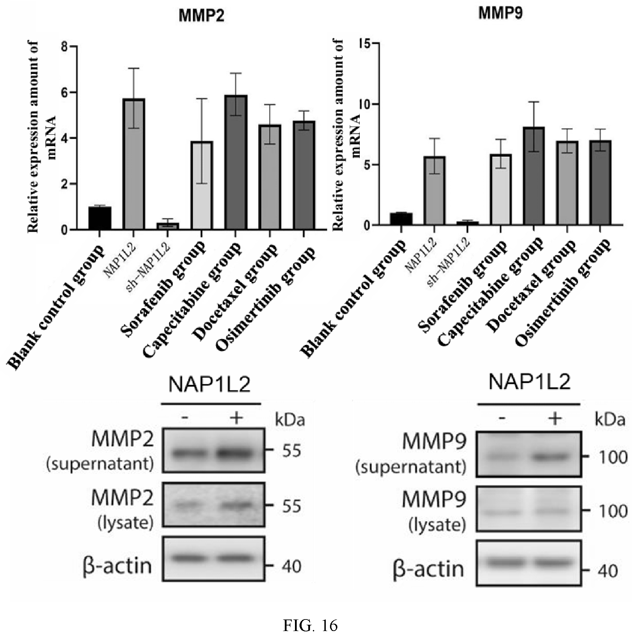

Results are shown in FIG. 16. After the recombinant NAP1L2 protein was added to the HaCaT keratinocytes, the mRNA and protein expression amounts of matrix metalloproteinases MMP2 and MMP9 were significantly increased; and when NAP1L2 siRNA was added to the HaCaT keratinocytes to inhibit the expression of NAP1L2, the mRNA and protein expression amounts of the matrix metalloproteinases MMP2 and MMP9 were significantly decreased. Numerous studies have confirmed that abnormal expression of matrix metalloproteinases is closely associated with various skin-related diseases (Kumper M. et al., Am. J. Physiol. Cell. Physiol. 2022, 323(4): 1290-1303).

### Example 6: Regulation of the expression of human keratinocyte differentiation markers by m6A

1*10⁸ HaCaT cells were seeded in culture dishes, into which a recombinant HBEGF protein (Thermo Fisher Scientific, Cat.#100-47-1mg) was added. The cells were placed in an incubator containing 5% carbon dioxide for culture at 37°C for 24 hours, into which an HBEGF antibody (Invitrogen, Cat.#406316) was added, or METTL3 siRNA (sh-METTL3, GeneChem) and NAP1L2 siRNA (sh-NAP1L2, GeneChem) were transfected. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the supernatant, and the protein expression amounts of keratinocyte differentiation markers KRT1, KRT10, Loricrin, and Involucrin were measured by Western blot.

Total RNA was extracted using Trizol reagent (Sigma Aldrich). 1 µg of RNA was reverse-transcribed into cDNA using a cDNA reverse transcription kit (Transgene Biotech, Cat.#AT311-03). 1.25 µL of a primer (with a primer sequence shown in Table 2), 10 µL of an iTag Universal SYBR Green supermix (Bio-Rad, Cat.#172-5125), and an appropriate amount of DEPC ultrapure water were added to formulate 20 µL of a reaction solution to perform an RT-PCR reaction. Upon completion of the reaction, the reaction solution was subjected to agarose gel electrophoresis to determine the mRNA expression level of the keratinocyte differentiation markers KRT1, KRT10, Loricrin, and Involucrin.

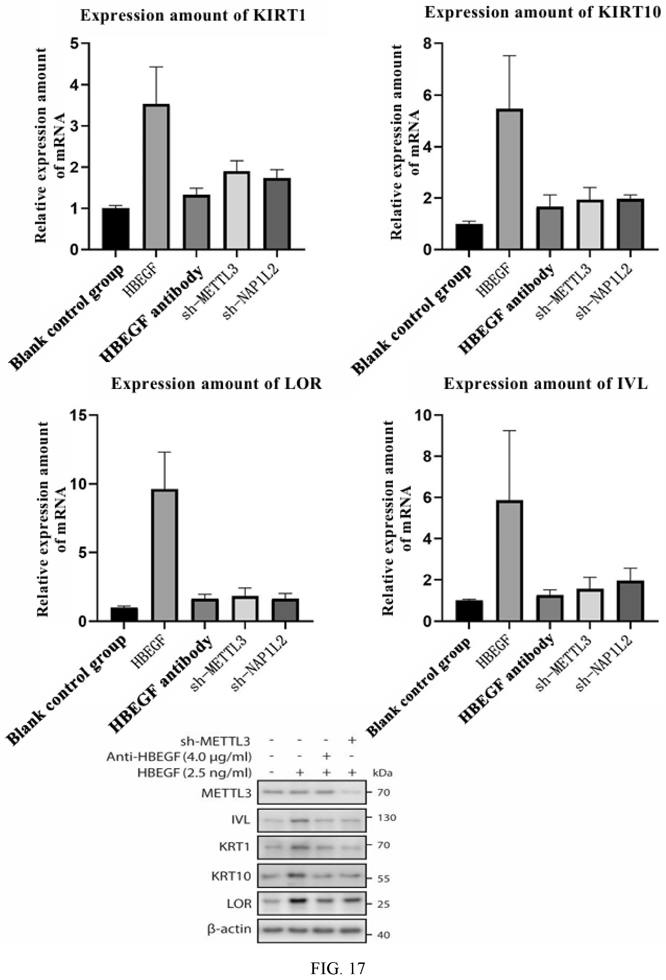

Results are shown in FIG. 17. After the recombinant HBEGF protein was added to the HaCaT keratinocytes, the mRNA and protein expression amounts of the keratinocyte differentiation markers KRT1, KRT10, Loricrin, and Involucrin were significantly increased, indicating that the HaCaT keratinocytes were in a highly differentiated state; however, when the HBEGF antibody or METTL3 or NAP1L2 siRNA was added to inhibit an m6A methylation function in the cells, the mRNA and protein expression amounts of the keratinocyte differentiation markers KRT1, KRT10, Loricrin, and Involucrin are significantly decreased. This indicates that m6A regulated the expression of the keratinocyte differentiation markers, thereby regulating the differentiation of the keratinocytes.

### Example 7: Impacts of compounds on an m6A methylation level of THP-1 cells

1*10⁸ THP-1 cells were seeded in culture dishes, into which 4 nM and 400 nM compound of formula (I), 4 nM and 4 µM nicotinamide, and 5 µM m6A methyltransferase inhibitor UZH2 were added, respectively. The cells were cultured in an incubator containing 5% CO₂ at 37°C for 24 hours. After discarding culture media and washing with PBS, the cells were centrifuged, and the supernatant was discarded. 400 µL of an RLT cell lysis buffer was added, and the cells were stored at -80°C. A portion of samples were selected, into which 400 µL of 70% ethanol was added and evenly stirred. 700 µL of a sample was transferred to an RNeasy centrifugation column and centrifuged for 15 seconds (8,000 g, 25°C). Then, 700 µL of an RW1 buffer solution was added and centrifuged for 15 seconds (8,000 g, 25°C), followed by the addition of 500 µL of an RPE buffer solution and centrifugation for 16 seconds (8,000 g, 25°C). The above steps were repeated twice, followed by centrifugation for 2 minutes to completely remove the eluent. 30 µL of nuclease-free water was added to a chromatographic column, and the cells were incubated for 5 minutes and then centrifuged for 2 minutes (12,000 g, 25°C). The steps were repeated 3 times. 2 µL of an RNA sample was selected and dissolved in 98 µL of a 10 mM Tris buffer solution, and the absorbance at 260 nm was measured using a Nanodrop to determine the RNA concentration.

50 µg of total RNA was selected and dissolved in 100 µL of nuclease-free water. Oligo (dT) magnetic beads were resuspended, 50 µL of the magnetic beads were transferred to a 1.5 mL test tube, 500 µL of a binding buffer solution was added, the mixture was allowed to stand, and the supernatant was removed. 100 µL of an RNA sample was added to 200 µL of a magnetic bead suspension for mixing, the mixture was stirred and incubated in a Thermomixer at 25°C for 5 minutes, the supernatant was discarded, and then 200 µL of a washing buffer solution was added. The steps were repeated 2 times, and then centrifugation was performed for 10 seconds (2,000 g, 25°C). 50 µL of an elution buffer solution was added and evenly mixed, the mixture was heated to 75°C, the supernatant was transferred to a new 1.5 mL test tube and purified using an RNA Clean & Concentrator kit, and the RNA concentration was determined.

20 µL (approximately 200 ng) of an mRNA sample and 20 µL of a Nuclease P1 digestion premix (including 0.5 µL of 2 unit/µL Nuclease P1, 0.4 µL of 5 M NaCl, 2 µL of 0.1 M ZnCl₂, and 17.1 µL of PCR-grade water) were added into each test tube, and the mixture was stirred and incubated in a Thermomixer at 37°C for 2 hours. 2 µL of a 2 M NH₄HCO₃ solution and 1 unit of alkaline phosphatase were added, and the mixture was stirred and incubated in a Thermomixer at 37°C for 2 hours. 1 µL of a 1.2 M HCl neutralization solution was added, and centrifugation was performed for 30 minutes (16,000 g, 4°C). 20 µL of the supernatant was collected, an m6A methylation fragment was analyzed by LC-MS, and the m6A inhibition rate was calculated. 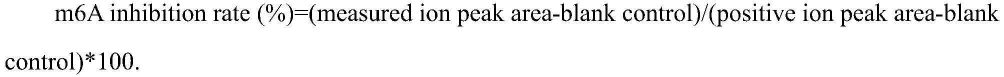 m6A inhibition rate (%)=(measured ion peak area-blank control)/(positive ion peak area-blank control)*100.

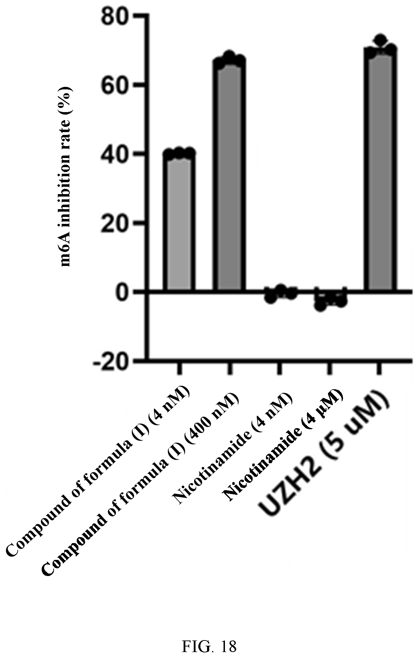

Results are shown in FIG. 18. The compound of formula (I) can significantly inhibit the m6A methylation level of mRNA of the cells in a concentration-dependent manner. At a concentration of 400 nM, the compound of formula (I) exhibited m6A inhibition activity comparable to that of the m6A methyltransferase inhibitor UZH2 at a concentration of 5 µM. However, nicotinamide exhibits no inhibitory effect on the m6A methylation level of mRNA of the cells at either a low concentration (4 nM) or a high concentration (4 µM).

### Example 8: Impacts of compounds on an m6A methylation level of human keratinocytes

HaCaT cells were cultured in DMEM culture medium containing 10% fetal bovine serum, 100 U/mL penicillin, and 100 µg/mL streptomycin. Culture dishes were placed in an incubator containing 5% CO₂ for culture at 37°C for 24 hours. An equal volume of DMSO was added in control group 1, 50 nM sorafenib, capecitabine, docetaxel, and osimertinib were respectively added in control groups A, B, C, and D, and 400 nM test compounds or nicotinamide were respectively added in sample groups A1, A2, B1, B2, C1, C2, D1, and D2 after the addition of 50 nM corresponding compounds. The cells were continuously cultured for 24 hours, washed with PBS, and then collected by high-speed centrifugation. Total RNA was extracted using Trizol reagent (Sigma Aldrich), and RNA m6A methylation of a sample was quantitatively detected using an EpiQuik m6A RNA methylation quantification kit. The concentrations of positive control samples for a standard curve were 0.01 ng/µl, 0.02 ng/µl, 0.05 ng/µl, 0.1 ng/µl, 0.2 ng/µl, and 0.5 ng/µl. The absorbance at 450 nm was read using a microplate reader (Tecan GENios). A quantitative calculation formula for m6A is as follows: $m 6 A \left(ng\right) = \left(absorbance of sample well-absorbance of background well\right) / slope of standard curve$ $m 6 A \left(%\right) = m 6 A \left(ng\right) / RNA amount of sample \left(ng\right) * 100 % .$

Results are shown in Table 1. The treatment with the sorafenib, the capecitabine, the docetaxel, and the osimertinib can significantly increase the m6A methylation level of human HaCaT keratinocytes (as shown in Table 1). The compound of formula (I) can effectively inhibit an abnormal increase in the RNA m6A level of human keratinocytes caused by chemotherapeutic drugs and multikinase inhibitors.

**Table 1 Fold changes in the m6A methylation level relative to control group 1. A specific calculation method is: fold change=measured m6A amount/measured m6A amount of control 1.**

| **Group** | **Drug/compound** | **m6A fold change** | **Group** | **Drug/compound** | **m6A fold change** |
|---|---|---|---|---|---|
| A | Sorafenib | 4.5 | B | Capecitabine | 6.7 |
| A1 | Compound of formula (I) | 1.4 | B1 | Compound of formula (I) | 1.1 |
| A2 | Nicotinamide | 4.7 | B2 | Nicotinamide | 4.5 |
| C | Docetaxel | 5.6 | D | Osimertinib | 4.8 |
| C1 | Compound of formula (I) | 1.0 | D1 | Compound of formula (I) | 1.2 |
| C2 | Nicotinamide | 5.4 | D2 | Nicotinamide | 5.0 |

### Example 9: Inhibitory effects of compounds on the differentiation of HaCaT keratinocytes

Human HaCaT keratinocytes were cultured in DMEM culture medium containing 10% fetal bovine serum, 100 U/mL penicillin, and 100 µg/mL streptomycin. The cells were seeded into a 96-well plate at a density of 2*10⁶ cells per square centimeter and placed in an incubator containing 5% carbon dioxide for culture at 37°C for 24 hours. An equal volume of DMSO was added into control well 1, a 2.5 ng/mL recombinant human HBEGF protein (Abcam, ab205523) was added into control well 2, and a 2.5 ng/mL recombinant human HBEGF protein and corresponding concentrations of test compounds were added into sample wells. The cells were continuously cultured for 24 hours, washed with PBS, and then collected by high-speed centrifugation. Total RNA was extracted using Trizol reagent (Sigma Aldrich). 1 µg of RNA was reverse-transcribed into cDNA using a cDNA reverse transcription kit (Transgene Biotech, AT311-03). 1.25 µL of a primer (with a primer sequence shown in Table 2), 10 µL of an iTag Universal SYBR Green supermix (Bio-Rad, 172-5125), and an appropriate amount of DEPC ultrapure water were added to formulate 20 µL of a reaction solution to perform an RT-PCR reaction. Upon completion of the reaction, the reaction solution was subjected to agarose gel electrophoresis to determine the mRNA expression level of keratinocyte differentiation markers KRT1, KRT10, Loricrin, and Involucrin.

**Table 2 Sequence information of some primers**

| | | |
|---|---|---|
| KRT1 | Forward primer | 5'-GTCAAGTCCTCTGGTGGCAG-3' |
| | Reverse primer | 5'-AAGGCTGGGACAAATCGACC-3' |
| KRT10 | Forward primer | 5'-CCCGGGTGTTGATCTGACTC-3' |
| | Reverse primer | 5'-CCAGGCTTCAGCATCTTTGC-3' |
| Loricrin | Forward primer | 5'-TCATGATGCTACCCGAGGTTTG-3' |
| | Reverse primer | 5'-CAGAACTAGATGCAGCCGGAGA-3' |
| Involucrin | Forward primer | 5'-TCGCTCCTCAAGACTGTTCCTCC-3' |
| | Reverse primer | 5'-CAGGCAGTCCCTTTACAGCA-3' |

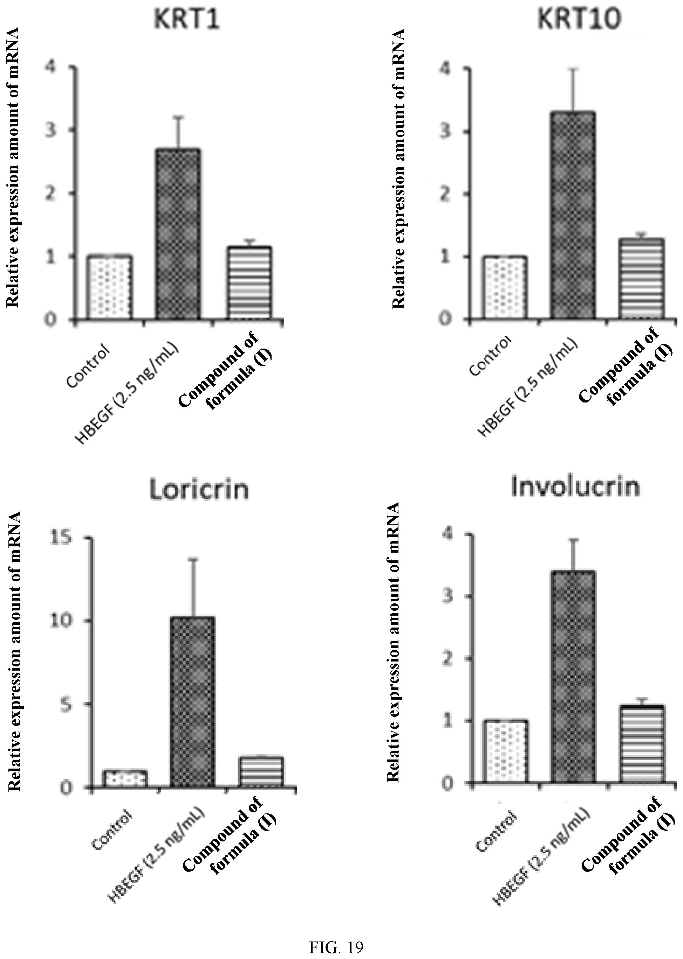

As shown in FIG. 19, after the recombinant human HBEGF protein was added for induction, the mRNA expression level of the HaCaT differentiation markers KRT1, KRT10, Loricrin, and Involucrin were significantly increased. However, after the compound of formula (I) was added, the mRNA expression level of the HaCaT differentiation markers KRT1, KRT10, Loricrin, and Involucrin were significantly inhibited.

### Example 10: Inhibitory effects of compounds on the differentiation of keratinocytes in rat models with hand-foot skin reaction

After adaptive feeding for one week, SD rats (with a body weight of approximately 200 g) were randomly divided into groups with balanced body weight, with 12 rats in each group. Modeling drugs (including sorafenib, erlotinib, afatinib, and osimertinib) were respectively dissolved in a solution containing 5% of DMSO, 45% of PEG400, and 50% of H₂O, and the modeling drugs were diluted to required concentrations and then administered once daily by gavage at doses shown in Table 3. After administration by gavage for 1 hour, 0.05 g of ointments containing different mass proportions of test compounds were evenly applied to left paws of the rats, a blank matrix ointment was applied to right paws of the rats to serve as a self-control, and no ointment was applied to the rats in a blank control group. After drug application, four limbs were continuously immobilized for 2 hours, then wiped with clean water to remove the residual drugs, removed from immobilization, and restored to free movement. The modeling drugs, the blank matrix ointment, and the ointments containing the test compounds were all administered once daily. After continuous administration for 30 days, the rats were euthanized, and paw plantar skin tissues were collected, fixed in 10% neutral formalin, cut into 5 µm sections, dehydrated, and then embedded in paraffin. The ointments for the test compounds were prepared by mixing the compounds, white beeswax, white petrolatum, and light liquid paraffin in specific ratios (weight ratios of 3 ointments were respectively 1:18:58:23, 3:18:57:22, and 10:20:40:30). The blank matrix ointment was prepared by mixing white beeswax, white petrolatum, and light liquid paraffin in a specific ratio (weight ratio of 18:60:22).

Tissue staining: After dewaxing and rehydration, the paw plantar skin tissue sections of the above rats were stained in a hematoxylin solution for several minutes, cleaned, and placed in 1% acid alcohol for soaking until the sections faded to light blue-red. After cleaning with running water for 5 minutes, the sections were stained with eosin for 2-3 minutes, cleaned to remove excess dyes, dehydrated, then permeabilized by adding xylene for several minutes, and sealed and fixed with a neutral resin. The stratum corneum was observed under an optical microscope, and the thickness of the epidermal stratum corneum was measured using Dmetrix software.

Immunohistochemical staining: After dewaxing and hydration, the paw plantar skin tissue sections of the above rats were incubated with 3% H₂O₂ at room temperature for 30 minutes and blocked with 10% goat serum for 30 minutes after antigen retrieval. A KRT1 antibody (Abcam, ab93652) and a Loricrin antibody (Abcam, ab183646) were added dropwise, the sections were incubated overnight at 4°C, and an HRP secondary antibody (ZSGB-BIO, PV-6001) and a DAB kit (ZSG-BIO, ZLI9017) were added for color development. The sections were re-stained with hematoxylin, cleaned, and sealed and fixed with a neutral resin, and the expression amounts of KRT1 and Loricrin were observed under an optical microscope.

Criteria for determining successful modeling of the above rat models are as follows: (i) symptoms, such as erythema, swelling, desquamation, ulcers, or blisters, occur at paw sites; and/or (ii) the stratum corneum thickness in tissue staining is significantly higher than that of normal rats; and/or (iii) markers, such as KRT1, KRT5, and Loricrin, are significant increased. An incidence rate calculation method involves a proportion of animals in each group meeting the above criteria for determining successful modeling, that is, incidence rate = (number of successfully modeled rats/total number of rats in the group)*100%.

Pathological scoring criteria for tissue staining of the above rats are as follows: no blisters, recorded as 0 point; the number of blisters being 1-3, recorded as 1 point; the number of blisters being 4-6, recorded as 2 points; the number of blisters being 7-9, recorded as 3 points; and the number of blisters being more than 10, recorded as 4 points; an inflammation area accounting for less than 10% of a total section area, recorded as 0 point; the inflammation area accounting for 10-25% of the total section area, recorded as 1 point; the inflammation area accounting for 25-50% of the total section area, recorded as 2 points; the inflammation area accounting for 50-75% of the total section area, recorded as 3 points; and the inflammation area accounting for more than 75% of the total section area, recorded as 4 points; an congestion area accounting for less than 10% of a total section area, recorded as 0 point; the congestion area accounting for 10-25% of the total section area, recorded as 1 point; the congestion area accounting for 25-50% of the total section area, recorded as 2 points; the congestion area accounting for 50-75% of the total section area, recorded as 3 points; and the congestion area accounting for more than 75% of the total section area, recorded as 4 points. Scoring was performed using a double-blind scoring system. After completion of statistics, data for each group was presented in the form of mean + SEM (N=12).

**Table 3 Doses of drugs used in rat models and experimental results**

| **Serial number** | **Modeling drug** | **Dose** | **Compound and its mass proportion in an ointment** | | **Incidence rate** | |
|---|---|---|---|---|---|---|
| | | | Left paw | Right paw | Left paw | Right paw |
| 1 | Sorafenib | 100 mg/kg | Compound of formula (I), 1% | None | 2/12 (17%) | 9/12 (75%) |
| 2 | Erlotinib | 70 mg/kg | Compound of formula (I), 1% | None | 3/12 (25%) | 10/12 (83%) |
| 3 | Afatinib | 50 mg/kg | Compound of formula (I), 1% | None | 3/12 (25%) | 9/12 (75%) |
| 4 | Osimertinib | 60 mg/kg | Compound of formula (I), 1% | None | 2/12 (17%) | 8/12 (67%) |
| 5 | Sorafenib | 100 mg/kg | Compound of formula (I), 3% | None | 2/12 (17%) | 4/12 (33%) |
| 6 | Erlotinib | 70 mg/kg | Compound of formula (I), 3% | None | 2/12 (17%) | 5/12 (42%) |
| 7 | Afatinib | 50 mg/kg | Compound of formula (I), 3% | None | 2/12 (17%) | 4/12 (33%) |
| 8 | Osimertinib | 60 mg/kg | Compound of formula (I), 3% | None | 2/12 (17%) | 4/12 (33%) |
| 9 | Sorafenib | 100 mg/kg | Compound of formula (I), 10% | None | 0/12 (0%) | 4/12 (33%) |
| 10 | Erlotinib | 70 mg/kg | Compound of formula (I), 10% | None | 1/12 (8%) | 4/12 (33%) |
| 11 | Afatinib | 50 mg/kg | Compound of formula (I), 10% | None | 0/12 (0%) | 3/12 (25%) |
| 12 | Osimertinib | 60 mg/kg | Compound of formula (I), 10% | None | 0/12 (0%) | 3/12 (25%) |
| 13 | Sorafenib | 100 mg/kg | None | None | 9/12 (75%) | |
| 14 | Erlotinib | 70 mg/kg | None | None | 10/12 (83.3%) | |
| 15 | Afatinib | 50 mg/kg | None | None | 8/12 (66.7%) | |
| 16 | Osimertinib | 60 mg/kg | None | None | 9/12 (75%) | |

As shown in Table 3, when the sorafenib, the erlotinib, the afatinib, and the osimertinib were respectively administered once daily by gavage at doses of 100 mg/kg, 70 mg/kg, 50 mg/kg, and 60 mg/kg to the SD rats, successful modeling rates for the hand-foot skin reaction were 75%, 83.3%, 66.7%, and 75%, respectively. The compound of formula (I) at a low dose (such as the compound at a mass content of 1%) can significantly decrease the incidence rate of hand-foot skin reaction at a drug application site. The compound of formula (I) at a high mass content (such as the compound at a mass content of 3% and 10%) can significantly decrease the incidence rate of hand-foot skin reaction in all paws of the rats.

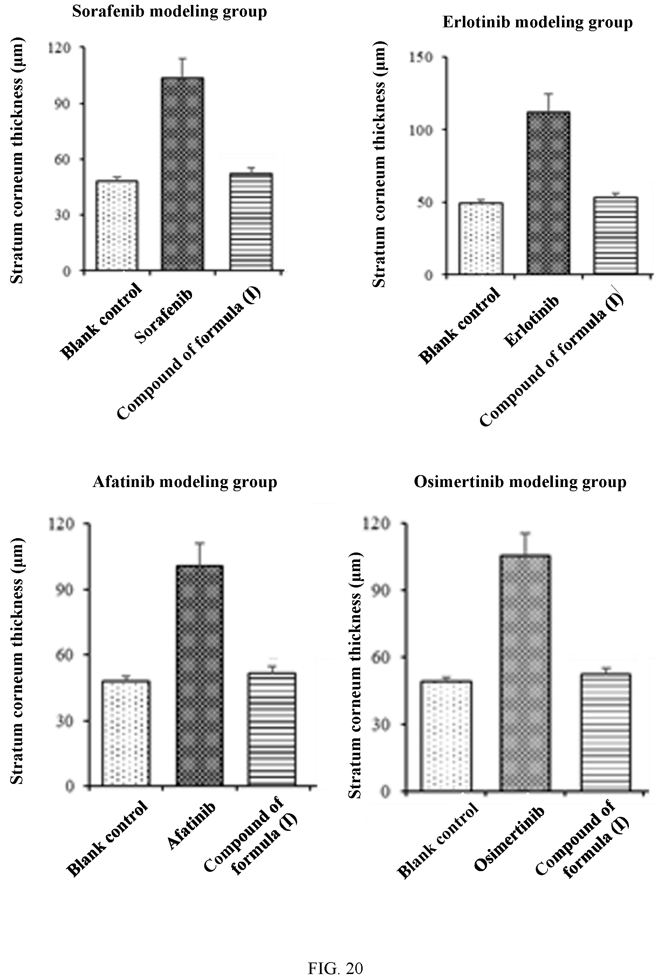

According to stratum corneum thickness measurement results shown in FIG. 20, in the successfully modeled rats using the sorafenib, the erlotinib, the afatinib, and the osimertinib, the stratum corneum thickness of the paw plantar skin was significantly higher than that of normal rats. The compound of formula (I) can effectively decrease the stratum corneum thickness of the paw plantar skin of the modeled rats.

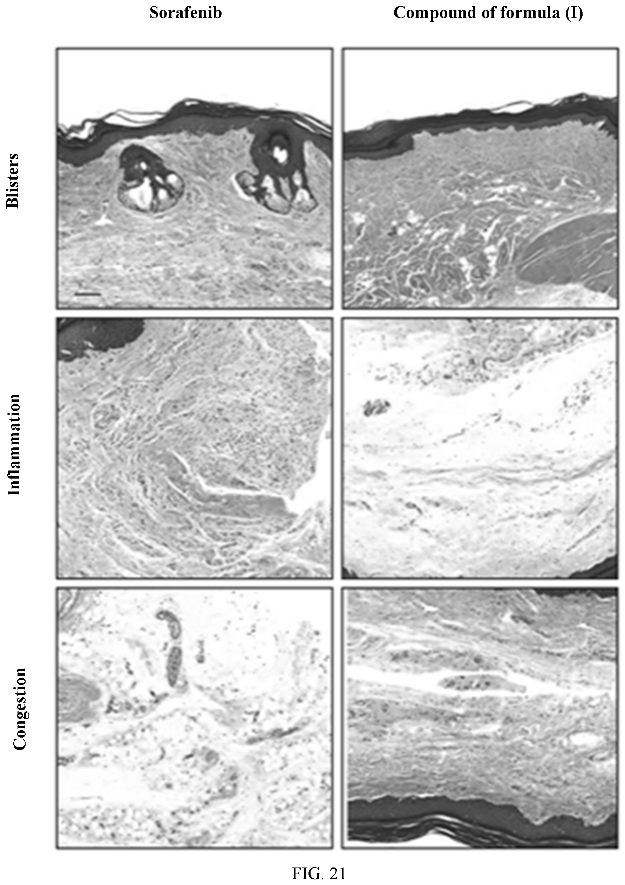

According to tissue staining results shown in FIG. 21, due to skin toxicity caused by the modeling drugs, blisters can be formed under the epidermis and in the epidermis of the paw plantar skin of the rats, inflammatory cell infiltration occurs in the dermis, and congestion symptoms occur in skin tissues. The compound of formula (I) can effectively alleviate the formation of blisters in the epidermis and subepidermis caused by the above modeling drugs, inhibit the inflammatory cell infiltration in the dermis, and significantly improve the skin congestion phenomenon.

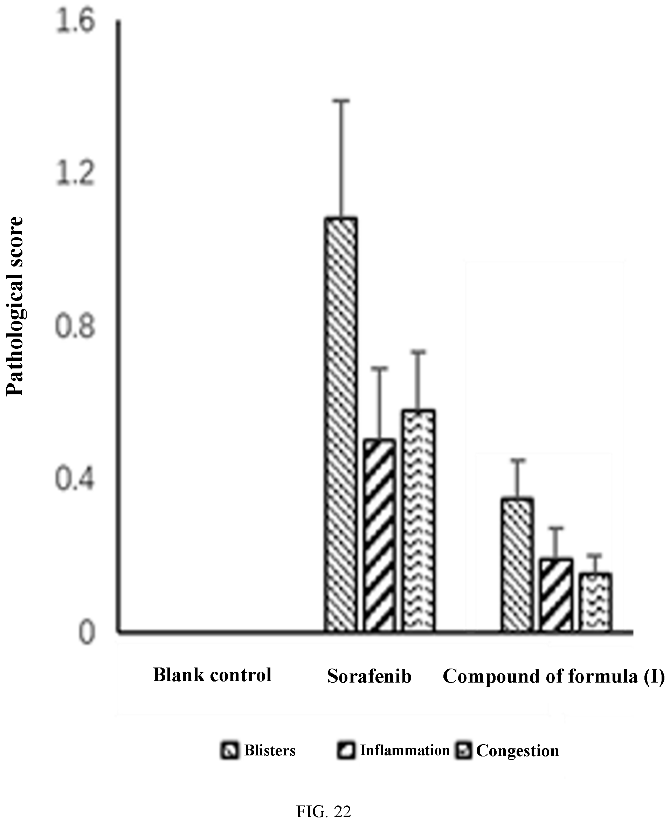

According to pathological scoring results shown in FIG. 22, the modeling drugs can cause the formation of blisters under the epidermis and in the epidermis at paw plantar sites of the rats, inflammatory cell infiltration occurs in the dermis, and congestion symptoms occur in skin tissues. The compound of formula (I) can effectively alleviate the formation of blisters in the epidermis and subepidermis caused by the above modeling drugs, inhibit the inflammatory cell infiltration in the dermis, and significantly alleviate the skin congestion phenomenon.

Immunohistochemical staining results also showed that in the successfully modeled rats using the sorafenib, the erlotinib, the afatinib, and the osimertinib, KRT1 and Loricrin were significantly increased in tissues. However, with the addition of compounds, KRT1 and Loricrin were significantly decreased in the tissues. In summary, the compound of formula (I) can effectively inhibit the differentiation of keratinocytes both *in vitro* and *in vivo*, thus exhibiting potential for treatment of a hyperkeratosis-related disease.

### Example 11: Effect of the compound of formula (I) in a chemotherapeutic drug-induced mouse model with hand-foot syndrome

After adaptive feeding for 7 days, SPF-grade ICR mice (male, 5-6 weeks old, with a body weight of approximately 35 g) were randomly divided into groups, including a blank control group (6 mice), a capecitabine modeling group (6 mice), a docetaxel modeling group (6 mice), a capecitabine + compound of formula (I) group (6 mice), a docetaxel + compound of formula (I) group (6 mice), a capecitabine + nicotinamide group (6 mice), and a docetaxel + nicotinamide group (6 mice). The groups were respectively administered corresponding modeling drugs by oral gavage (capecitabine at 200 mg/kg and docetaxel at 25 mg/kg) once daily; and the blank control group was administered an equivalent volume of normal saline. After administration with the chemotherapeutic drugs for 2 weeks, the ointments containing the compound of formula (I) (at a compound content of 3%) used in Example 10 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind feet of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of Na₂HPO₄, and 1 M HCl, pH 7.4). The sections were permeabilized with 0.5% Triton-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

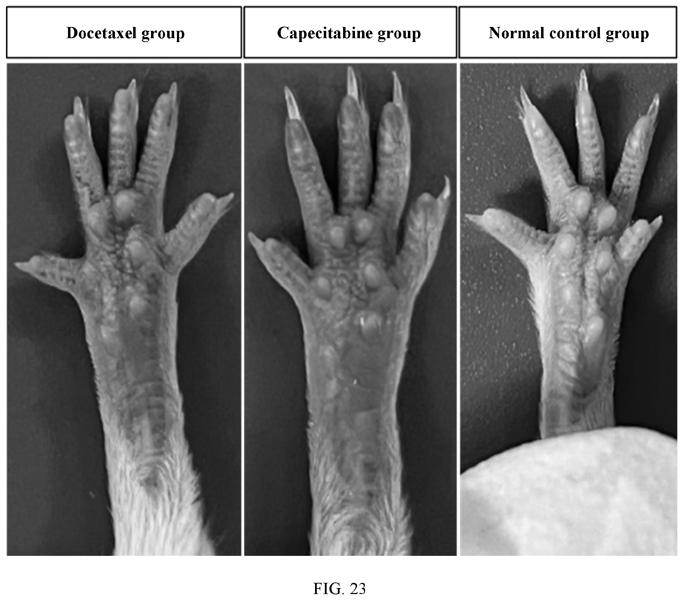

Mouse skin appearance results are shown in FIG. 23. After modeling with the capecitabine and the docetaxel for 14 days, the skin of the hind limb toes of the mice had obvious lesions. Compared with the normal control group, the skin of the hind limb toes of the mice in the capecitabine group and the docetaxel group had obvious erythema, rhagades, and blisters.

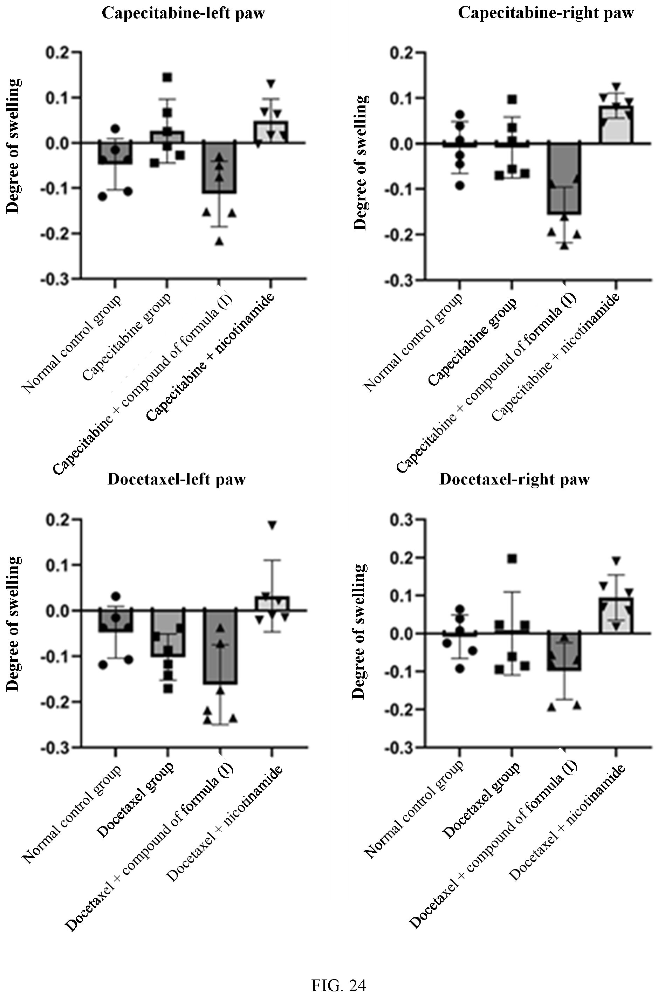

The degree of swelling of the hind limb toes of the mice is shown in FIG. 24. After continuous oral administration of the modeling drugs including the capecitabine and the docetaxel for 14 days, swelling of the hind limb toes of the mice was caused. Topical application of the ointments containing the compound of formula (I) to the hind limb toes of the mice can significantly alleviate the swelling of the toes caused by the modeling drugs (p<0.05). However, it was not observed that oral administration of nicotinamide at 100 mg/kg significantly alleviated the swelling of the hind limb toes of the mice.

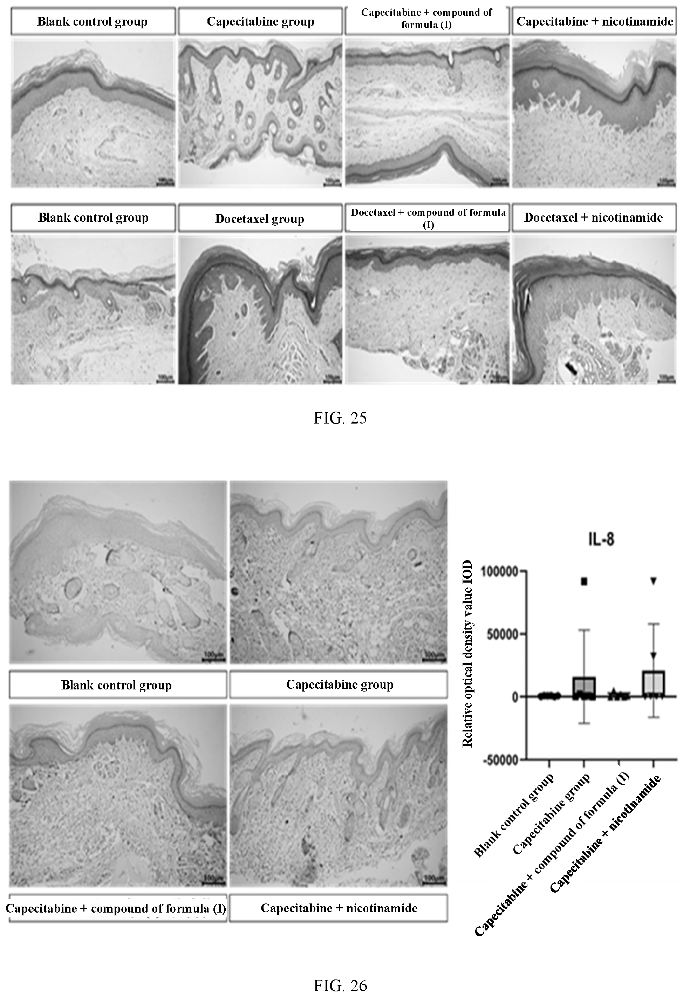

Pathological staining results of toe skin tissues are shown in FIG. 25. Compared with the normal control group, after continuous oral administration of the modeling drugs including the capecitabine and the docetaxel for 14 days, the epidermal layer and the stratum corneum in the skin tissues of the hind limb toes of the mice were significantly thickened, with increased granular cells in the basal layer and infiltration of inflammatory cells observed. Topical application of the ointments containing the compound of formula (I) to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of nicotinamide at 100 mg/kg mildly alleviated the thickening of the epidermal layer and the stratum corneum in the skin tissues of the hind limb toes of the mice, with slightly increased granular cells in the basal layer and infiltration of inflammatory cells still observed.

Immunohistochemical results of the capecitabine modeling series groups are shown in FIG. 26. Compared with the normal control group, after continuous oral administration of the modeling drug capecitabine for 14 days, cytokine IL-8 in the skin tissues of the hind limb toes of the mice was significantly increased (p<0.05). This indicates that oral administration of the capecitabine resulted in skin inflammation of the mice, which is consistent with a clinical trial observation result. Topical application of the ointments containing the compound of formula (I) to the hind limb toes of the mice can significantly decrease the expression level of cytokine IL-8 in the skin tissues of the hind limb toes of the mice (p<0.05). However, oral administration of the nicotinamide at 100 mg/kg didn't have a significant impact on the expression level of cytokine IL-8 in the skin tissues of the hind limb toes of the mice.

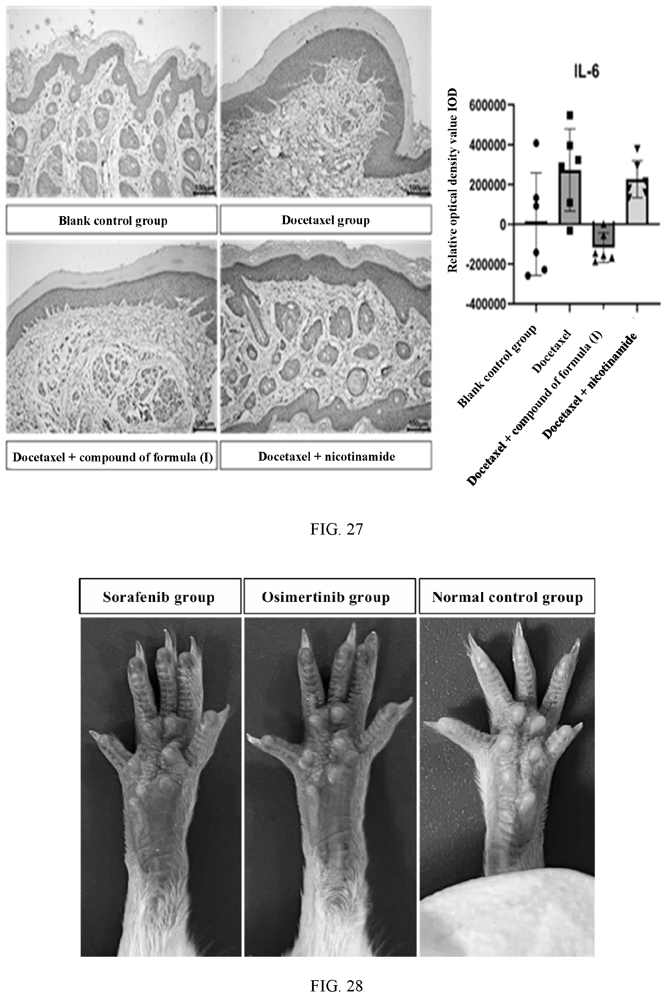

Immunohistochemical results of the docetaxel modeling series groups are shown in FIG. 27. Compared with the normal control group, after continuous oral administration of the modeling drug docetaxel for 14 days, cytokine IL-6 in the skin tissues of the hind limb toes of the mice was significantly increased (p<0.05). This indicates that oral administration of the docetaxel resulted in skin inflammation of the mice, which is consistent with a clinical trial observation result. Topical application of the ointments containing the compound of formula (I) to the hind limb toes of the mice can significantly decrease the expression level of cytokine IL-6 in the skin tissues of the hind limb toes of the mice (p<0.05). However, oral administration of the nicotinamide at 100 mg/kg didn't have a significant impact on the expression level of cytokine IL-6 in the skin tissues of the hind limb toes of the mice.

### Example 12: Effect of the compound of formula (I) in a kinase inhibitor-induced mouse model with hand-foot skin reaction

After adaptive feeding for 7 days, SPF-grade ICR mice (male, 5-6 weeks old, with a body weight of approximately 35 g) were randomly divided into groups, including a blank control group (6 mice), a sorafenib modeling group (6 mice), an osimertinib modeling group (6 mice), a sorafenib + compound of formula (I) group (6 mice), an osimertinib + compound of formula (I) group (6 mice), a sorafenib + nicotinamide group (6 mice), and an osimertinib + nicotinamide group (6 mice). The groups were respectively administered corresponding modeling drugs by oral gavage (sorafenib at 100 mg/kg and osimertinib at 10 mg/kg) once daily; and the blank control group was administered an equivalent volume of normal saline. After administration with the chemotherapeutic drugs for 2 weeks, the ointments containing the compound of formula (I) (at a compound content of 3%) used in Example 10 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind feet of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of Na₂HPO₄, and 1 M HCl, pH 7.4). The sections were permeabilized with 0.5% Triton-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

Results are shown in FIG. 28. After modeling with the sorafenib and the osimertinib for 14 days, the skin of the hind limb toes of the mice had obvious lesions. Compared with the normal control group, the skin of the hind limb toes of the mice in the sorafenib group and the osimertinib group had obvious erythema and rhagades.

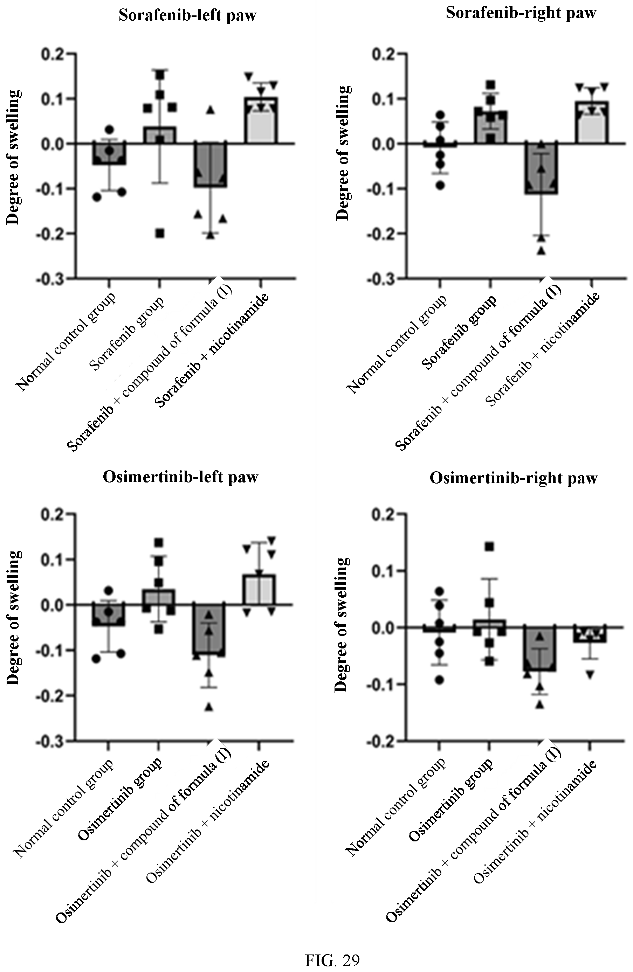

The degree of swelling of the hind limb toes of the mice is shown in FIG. 29. After continuous oral administration of the modeling drugs including the sorafenib and the osimertinib for 14 days, significant swelling of the hind limb toes of the mice was caused. Topical application of the ointments containing the compound of formula (I) to the hind limb toes of the mice can effectively alleviate the swelling of the toes caused by the modeling drugs. However, it was not observed that oral administration of the nicotinamide at 100 mg/kg significantly alleviated the swelling of the hind limb toes of the mice.

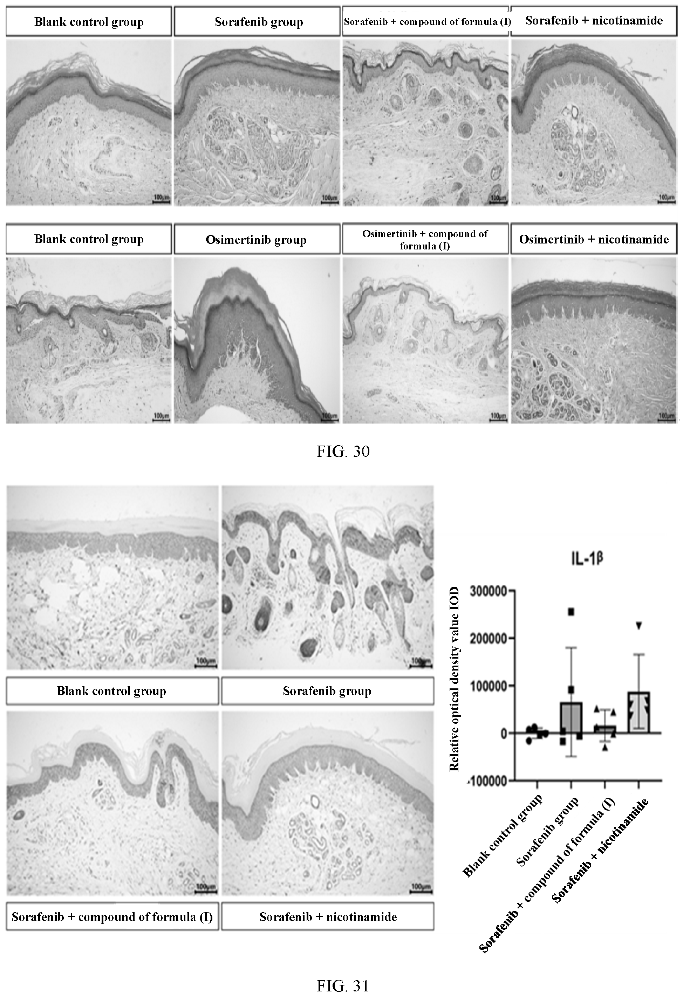

Pathological staining results of toe skin tissues are shown in FIG. 30. Compared with the normal control group, after continuous oral administration of the modeling drugs including the sorafenib and the osimertinib for 14 days, the epidermal layer and the stratum corneum in the skin tissues of the hind limb toes of the mice were significantly thickened, with increased granular cells in the basal layer and infiltration of inflammatory cells observed. Topical application of the ointments containing the compound of formula (I) to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of the nicotinamide at 100 mg/kg didn't significantly alleviate the thickening of the epidermal layer and the stratum corneum in the skin tissues of the hind limb toes of the mice, with slightly restored morphology of basal layer cells and infiltration of inflammatory cells still observed.

Immunohistochemical results of the sorafenib modeling series groups are shown in FIG. 31. Compared with the normal control group, after continuous oral administration of the modeling drug sorafenib for 14 days, cytokine IL-1β in the skin tissues of the hind limb toes of the mice was significantly increased (p<0.05). This indicates that oral administration of the sorafenib resulted in skin inflammation of the mice, which is consistent with a clinical trial observation result. Topical application of the ointments containing the compound of formula (I) to the hind limb toes of the mice can significantly decrease the expression level of cytokine IL-1β in the skin tissues of the hind limb toes of the mice (p<0.05). However, oral administration of the nicotinamide at 100 mg/kg didn't have a significant impact on the expression level of cytokine IL-1β in the skin tissues of the hind limb toes of the mice.

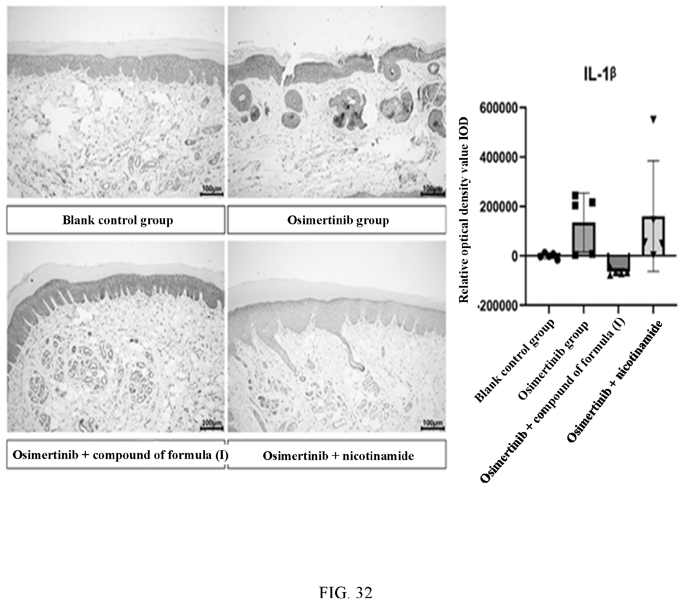

Immunohistochemical results of the osimertinib modeling series groups are shown in FIG. 32. Compared with the normal control group, after continuous oral administration of the modeling drug osimertinib for 14 days, cytokine IL-1β in the skin tissues of the hind limb toes of the mice was significantly increased (p<0.05). This indicates that oral administration of the osimertinib resulted in skin inflammation of the mice, which is consistent with a clinical trial observation result. Topical application of the ointments containing the compound of formula (I) to the hind limb toes of the mice can significantly decrease the expression level of cytokine IL-1β in the skin tissues of the hind limb toes of the mice (p<0.05). However, oral administration of the nicotinamide at 100 mg/kg didn't have a significant impact on the expression level of cytokine IL-1β in the skin tissues of the hind limb toes of the mice.

### Example 13: Preparation of a pharmaceutical co-crystal formed by the compound of formula (I) and isonicotinamide

Approximately 20 mg of the compound of formula (I) and 10.2 mg of isonicotinamide were weighed and placed in a 2 mL glass vial, and ethanol (0.2 mL) was added to obtain a suspension. The resulting sample was suspended and stirred at 5°C for 3 days. The resulting suspension was allowed to pass through a 0.45 µm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the isonicotinamide, with an XRPD pattern as shown in FIG. 1 and positions of characteristic peaks as shown in Table 4. A DSC spectrogram showed a melting Tₒₙₛₑₜ at 160.45°C. A stoichiometric ratio of the resulting pharmaceutical co-crystal was 1:1.

**Table 4 XRPD diffraction peak data of the pharmaceutical co-crystal formed by the compound of formula (I) and the isonicotinamide**

| 2θ(°) | D(Å) | Relative intensity (%) |
|---|---|---|
| 6.139 | 14.3846 | 1.90 |
| 9.386 | 9.41521 | 13.80 |
| 11.847 | 7.46425 | 29.10 |
| 13.641 | 6.48624 | 39.00 |
| 14.019 | 6.31196 | 87.70 |
| 15.097 | 5.86363 | 13.90 |
| 16.746 | 5.29 | 6.30 |
| 17.556 | 5.04765 | 8.30 |
| 17.893 | 4.9532 | 8.70 |
| 18.822 | 4.71099 | 60.00 |
| 19.428 | 4.56536 | 4.00 |
| 21.196 | 4.1883 | 100.00 |
| 21.875 | 4.05976 | 8.30 |
| 23.762 | 3.74148 | 18.30 |
| 24.300 | 3.65983 | 31.30 |
| 26.354 | 3.37908 | 10.30 |
| 26.566 | 3.3526 | 50.30 |
| 27.454 | 3.24611 | 5.00 |
| 28.722 | 3.10567 | 5.20 |
| 31.980 | 2.79636 | 4.40 |
| 33.296 | 2.68876 | 11.10 |
| 34.680 | 2.58452 | 5.00 |
| 35.936 | 2.49706 | 17.30 |

### Example 14: Preparation of a pharmaceutical co-crystal formed by the compound of formula (I) and glycolic acid

Approximately 20 mg of the compound of formula (I) and 6.3 mg of glycolic acid were weighed and placed in a 2 mL glass vial, and isopropyl acetate (0.2 mL) was added to obtain a suspension. The resulting sample was suspended and stirred at 5°C for 3 days. The resulting suspension was allowed to pass through a 0.45 µm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the glycolic acid, with an XRPD pattern as shown in FIG. 4 and positions of characteristic peaks as shown in Table 5. A DSC spectrogram showed a melting Tₒₙₛₑₜ @ 135.68°C. A stoichiometric ratio of the resulting pharmaceutical co-crystal was 1:1.

**Table 5 XRPD diffraction peak data of the pharmaceutical co-crystal formed by the compound of formula (I) and the glycolic acid**

| 2θ(°) | D(Å) | Relative intensity (%) |
|---|---|---|
| 10.588 | 8.34856 | 69.90 |
| 12.575 | 7.0336 | 42.90 |
| 14.928 | 5.92986 | 13.40 |
| 17.645 | 5.02244 | 67.40 |
| 18.231 | 4.8621 | 21.40 |
| 18.444 | 4.80666 | 17.70 |
| 19.706 | 4.50151 | 21.50 |
| 20.417 | 4.34628 | 12.20 |
| 21.232 | 4.18118 | 57.00 |
| 21.527 | 4.12465 | 72.50 |
| 23.125 | 3.84307 | 100.00 |
| 24.776 | 3.59063 | 38.50 |
| 25.300 | 3.51742 | 42.20 |
| 25.928 | 3.43368 | 9.80 |
| 26.357 | 3.37878 | 8.00 |
| 26.840 | 3.31902 | 15.00 |
| 27.608 | 3.22841 | 45.90 |
| 28.937 | 3.0831 | 20.80 |
| 30.116 | 2.96504 | 12.00 |
| 30.376 | 2.94025 | 20.60 |
| 32.070 | 2.78869 | 13.20 |
| 33.925 | 2.64028 | 19.70 |
| 35.159 | 2.55039 | 11.30 |
| 35.368 | 2.53583 | 10.60 |
| 35.719 | 2.51172 | 4.80 |
| 36.010 | 2.49209 | 13.70 |
| 36.881 | 2.43519 | 8.80 |
| 37.253 | 2.4117 | 9.10 |
| 38.478 | 2.33774 | 7.40 |
| 39.512 | 2.27891 | 7.90 |

### Example 15: Preparation of a pharmaceutical co-crystal formed by the compound of formula (I) and L-proline

Approximately 20 mg of the compound of formula (I) and 9.7 mg of L-proline were weighed and placed in a 2 mL glass vial, and ethanol (0.25 mL) was added to obtain a suspension. The resulting sample was suspended and stirred at 5°C for 3 days. The resulting suspension was allowed to pass through a 0.45 µm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the L-proline, with an XRPD pattern as shown in FIG. 7 and positions of characteristic peaks as shown in Table 6. A DSC spectrogram showed a melting Tₒₙₛₑₜ @ 183.55°C. A stoichiometric ratio of the resulting pharmaceutical co-crystal was 1:1.

**Table 6 XRPD diffraction peak data of the pharmaceutical co-crystal formed by the compound of formula (I) and the L-proline**

| 2θ(°) | D(Å) | Relative intensity (%) |
|---|---|---|
| 6.586 | 13.4092 | 2.00 |
| 7.120 | 12.4061 | 5.80 |
| 10.302 | 8.57947 | 100.00 |
| 13.153 | 6.72557 | 10.00 |
| 14.229 | 6.21936 | 22.20 |
| 14.871 | 5.95232 | 39.60 |
| 15.763 | 5.61748 | 1.70 |
| 16.443 | 5.38677 | 1.10 |
| 18.719 | 4.73667 | 1.50 |
| 19.762 | 4.48885 | 15.80 |
| 20.113 | 4.41121 | 3.20 |
| 20.643 | 4.2992 | 53.80 |
| 21.217 | 4.18422 | 1.30 |
| 21.383 | 4.15208 | 1.10 |
| 21.899 | 4.05545 | 2.50 |
| 22.829 | 3.89225 | 4.20 |
| 23.211 | 3.8291 | 14.00 |
| 23.611 | 3.7651 | 6.70 |
| 24.012 | 3.70312 | 2.30 |
| 24.982 | 3.56148 | 2.20 |
| 25.287 | 3.51915 | 2.90 |
| 26.465 | 3.36512 | 7.30 |
| 26.627 | 3.34511 | 3.70 |
| 27.860 | 3.19976 | 1.00 |
| 28.300 | 3.15105 | 2.60 |
| 28.621 | 3.11643 | 10.20 |
| 28.687 | 3.10934 | 3.40 |
| 30.305 | 2.94693 | 3.20 |
| 31.158 | 2.86817 | 9.40 |
| 31.789 | 2.81271 | 4.10 |
| 32.728 | 2.73405 | 1.40 |
| 33.199 | 2.69639 | 2.70 |
| 35.727 | 2.51117 | 2.50 |
| 36.432 | 2.46414 | 4.10 |
| 37.176 | 2.41656 | 8.20 |
| 38.026 | 2.36448 | 4.00 |

### Example 16: Preparation of a pharmaceutical co-crystal formed by the compound of formula (I) and L-proline

Approximately 300 mg of the compound of formula (I) and 145.8 mg of L-proline (1.0 equivalent) were weighed and placed in a 20 mL glass vial. 3.75 mL of ethanol was added, and the mixture was stirred at 5°C for 2 minutes to obtain a suspension. Approximately 5 mg of a seed crystal of a pharmaceutical co-crystal formed by the compound of formula (I) and L-proline was added to the above suspension. Then, 3 mL of ethanol was added to the above suspension and stirred at 5°C for 4 days. A solid portion was collected by centrifugation, and the obtained solid was dried under vacuum conditions at room temperature for about 2.5 hours. A total of 250 mg of a white powder was obtained, with a yield of 56%. X-ray powder diffraction detection showed that the product was the pharmaceutical co-crystal formed by the compound of formula (I) and the L-proline.

### Example 17: Preparation of a pharmaceutical co-crystal formed by the compound of formula (I) and nicotinamide

Approximately 20 mg of the compound of formula (I) and 10.3 mg of nicotinamide were weighed and placed in a 2 mL glass vial, and acetonitrile (0.25 mL) was added to obtain a suspension. The resulting sample was suspended and stirred at 5°C for 3 days. The resulting suspension was allowed to pass through a 0.45 µm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the nicotinamide, with an XRPD pattern as shown in FIG. 10 and positions of characteristic peaks as shown in Table 7. A DSC spectrogram showed a melting Tₒₙₛₑₜ @ 156.75°C. A stoichiometric ratio of the resulting pharmaceutical co-crystal was 1:1.

**Table 7 XRPD diffraction peak data of the pharmaceutical co-crystal formed by the compound of formula (I) and the nicotinamide**

| 2θ(°) | D(Å) | Relative intensity (%) |
|---|---|---|
| 7.020 | 12.5824 | 0.80 |
| 8.001 | 11.0416 | 0.80 |
| 9.860 | 8.96307 | 23.80 |
| 11.529 | 7.66899 | 9.10 |
| 13.311 | 6.64637 | 48.70 |
| 14.036 | 6.30468 | 2.30 |
| 16.007 | 5.53235 | 7.40 |
| 17.017 | 5.20623 | 62.70 |
| 17.503 | 5.06265 | 6.30 |
| 18.433 | 4.80945 | 2.30 |
| 19.752 | 4.49118 | 100.00 |
| 20.856 | 4.25583 | 32.30 |
| 21.661 | 4.09935 | 10.10 |
| 22.122 | 4.01509 | 1.80 |
| 23.161 | 3.83715 | 14.10 |
| 23.638 | 3.76081 | 74.50 |
| 23.887 | 3.72224 | 6.80 |
| 24.147 | 3.68278 | 22.00 |
| 24.503 | 3.63001 | 7.10 |
| 24.780 | 3.59009 | 12.50 |
| 25.671 | 3.46747 | 1.50 |
| 26.165 | 3.40303 | 12.80 |
| 26.405 | 3.37275 | 4.50 |
| 26.573 | 3.35172 | 43.30 |
| 27.028 | 3.29633 | 18.90 |
| 27.215 | 3.27408 | 4.70 |
| 28.866 | 3.09047 | 4.90 |
| 29.289 | 3.04678 | 1.60 |
| 29.791 | 2.9966 | 1.40 |
| 30.207 | 2.95624 | 2.30 |
| 30.766 | 2.90383 | 14.30 |
| 31.486 | 2.83909 | 4.20 |
| 31.723 | 2.81837 | 5.90 |
| 32.284 | 2.77071 | 9.90 |
| 32.820 | 2.72664 | 18.90 |
| 33.238 | 2.69328 | 5.30 |
| 33.622 | 2.66341 | 7.70 |
| 34.379 | 2.6065 | 3.70 |
| 34.795 | 2.57628 | 1.40 |
| 35.138 | 2.5519 | 1.90 |
| 35.999 | 2.49282 | 3.00 |
| 36.741 | 2.44414 | 16.80 |
| 37.087 | 2.42216 | 2.90 |
| 37.911 | 2.3714 | 1.40 |
| 38.328 | 2.34651 | 1.50 |
| 38.591 | 2.33115 | 5.50 |
| 38.665 | 2.32686 | 1.30 |

### Example 18: Preparation of a pharmaceutical co-crystal formed by the compound of formula (I) and nicotinamide

Approximately 300 mg of the compound of formula (I) and 154.6 mg of nicotinamide (1.0 equivalent) were weighed and placed in a 20 mL glass vial. 3.75 mL of acetonitrile was added, and the mixture was stirred at 5°C for 2 minutes to obtain a suspension. Approximately 5 mg of a seed crystal of a pharmaceutical co-crystal formed by the compound of formula (I) and nicotinamide was added to the above suspension. Then, 2 mL of acetonitrile was added to the above suspension and stirred at 5°C for 4 days. A solid portion was collected by centrifugation, and the obtained solid was dried under vacuum conditions at room temperature for about 2.5 hours. A total of 365 mg of a white powder was obtained, with a yield of 80%. X-ray powder diffraction detection showed that the product was the pharmaceutical co-crystal formed by the compound of formula (I) and the nicotinamide.

In summary, a total of 4 types of pharmaceutical co-crystals were obtained, where the glycolic acid co-crystal usually has a hygroscopicity problem, the isonicotinamide co-crystal was similar to the nicotinamide co-crystal. However the nicotinamide is a GRAS reagent (Generally Recognized As Safe), and the isonicotinamide is a non-GRAS reagent. Finally, the L-proline co-crystal and the nicotinamide co-crystal were selected for continuous studies on pharmaceutical properties such as stability and hygroscopicity.

### Example 19: Stability test of the pharmaceutical co-crystal formed by the compound of formula (I) and the L-proline and the pharmaceutical co-crystal formed by the compound of formula (I) and the nicotinamide

Open containers containing the pharmaceutical co-crystal formed by the compound of formula (I) and the L-proline and the pharmaceutical co-crystal formed by the compound of formula (I) and the nicotinamide were respectively placed at 25°C/92.5% RH for one week. Closed containers containing the pharmaceutical co-crystal formed by the compound of formula (I) and the L-proline and the pharmaceutical co-crystal formed by the compound of formula (I) and the nicotinamide were respectively placed at 60°C for one week. The samples after solid stability evaluation were characterized by XRPD and HPLC, and color change situations of the samples were observed. Results are shown in Table 8.

**Table 8 Solid stability of the pharmaceutical co-crystals formed by the compound of formula (I)**

| Experiment number | | Solid form | | | |
|---|---|---|---|---|---|
| | Crystal form | Pharmaceutical co-crystal formed by the compound of formula (I) and the L-proline | | Pharmaceutical co-crystal formed by the compound of formula (I) and the nicotinamide | |
| | Initial purity (HPLC) | 99.9% | | 99.8% | |
| | Initial color | White | | White | |
| | | Purity | Color change | Purity | Color change |
| BS1 | Solid, 25°C/92%RH, open condition, 1 week | | | | |
| | Solid (HPLC) | 99.8% | No color change | 99.7% | No color change |
| | Solid (XRPD) | Partially dissociated into a free crystal form and L-proline | | No crystal form transformation | |
| BS2 | Solid, 60°C, closed container, 1 week | | | | |
| | Solid (HPLC) | 99.8% | No color change | 99.7% | No color change |
| | Solid (XRPD) | No crystal form transformation | | No crystal form transformation | |

It can be known from Table 8 that after being placed in the open containers at 25°C/92%RH for one week or placed in the closed containers at 60°C for one week, both the pharmaceutical co-crystal formed by the compound of formula (I) and the L-proline and the pharmaceutical co-crystal formed with the nicotinamide showed no obvious decrease in purity. However, the pharmaceutical co-crystal formed by the compound of formula (I) and the L-proline was partially dissociated into a free crystal form and L-proline at 25°C/92.5%RH. The pharmaceutical co-crystal formed by the compound of formula (I) and the nicotinamide remained relatively stable under both conditions.

### Example 20: Solubility test of the pharmaceutical co-crystal formed by the compound of formula (I) and the L-proline and the pharmaceutical co-crystal formed by the compound of formula (I) and the nicotinamide

15.1 mg of the pharmaceutical co-crystal formed by the compound of formula (I) and the L-proline and 14.9 mg of the pharmaceutical co-crystal formed by the compound of formula (I) and the nicotinamide were accurately weighed and respectively placed in 20 mL glass vials. 5 mL of a vehicle was added into each vial. The mass of the compound of formula (I) in the weighed co-crystals was comparable to 10 mg of a free anhydrous crystal form. The resulting suspension was stirred at 37°C at a rotation speed of 400 rpm, and sampling was performed at 0.5 hour and 2 hours, respectively. Samples were centrifuged at 37°C at a rotation speed of 14,000 rpm for 5 minutes. The concentration of a supernatant was determined by HPLC, and the pH value of the supernatant was measured using a pH meter. Whether a residual solid (wet sample) had crystal form transformation after 2 hours was detected by XRPD. Experimental results are shown in Table 9.

**Table 9 Solid solubility of the pharmaceutical co-crystals formed by the compound of formula (I)**

| Experimental number | Solubility at 37°C; target concentration at 2 mg/mL (calculated as a free form); equilibrium for 2 hours; LOQ: 0.3 µg/mL | | | | | | |
|---|---|---|---|---|---|---|---|
| | | Physical form | | | | | |
| | | Pharmaceutical co-crystal formed by the compound of formula (I) and the L-proline, anhydrous crystal form | | | Pharmaceutical co-crystal formed by the compound of formula (I) and the nicotinamide, anhydrous crystal form | | |
| | Vehicle | Solubility (µg/mL) | | XRPD | Solubility (µg/mL) | | XRPD |
| | | 0.5h | 2h (pH) | | 0.5h | 2h (pH) | |
| ES1 | Water | 47.1 | 45.0 (7.5) | Free crystal form | 63.0 | 51.3 (8.0) | Free crystal form |
| ES2 | pH 4.5 acetate buffer solution (50 mM) | 39.5 | 23.7 (4.5) | Free crystal form | 39.3 | 27.8 (4.6) | Free crystal form |
| ES3 | pH 6.8 phosphate buffer solution (50 mM) | 46.2 | 43.7 (6.8) | Free crystal form | 60.7 | 52.7 (6.8) | Free crystal form |
| ES4 | pH 10.0 alkaline borate buffer solution | 46.1 | 45.9 (9.8) | Free crystal form | 60.8 | 54.7 (9.9) | Free crystal form |

It can be known from Table 9 that the pharmaceutical co-crystal formed by the compound of formula (I) and the L-proline as well as the pharmaceutical co-crystal formed with the nicotinamide were both converted into a free crystal form of the compound of formula (I) in four different vehicles. Furthermore, the pharmaceutical co-crystal formed with the L-proline and the pharmaceutical co-crystal formed with the nicotinamide both had higher solubility in the remaining vehicles, and the pharmaceutical co-crystal formed by the compound of formula (I) and the nicotinamide had higher solubility after dissolution for 2 hours in four vehicles than the pharmaceutical co-crystal formed with the L-proline.

### Example 21: Hygroscopicity test of the pharmaceutical co-crystal formed by the compound of formula (I) and the L-proline and the pharmaceutical co-crystal formed by the compound of formula (I) and the nicotinamide

At 25°C, water absorption and dehydration behaviors of the pharmaceutical co-crystal formed by the compound of formula (I) and the L-proline and the pharmaceutical co-crystal formed by the compound of formula (I) and the nicotinamide were studied by DVS testing. A DVS cycle was 40-0-95-0-40% RH. XRPD detection was performed on the samples after the DVS testing to determine whether crystal form transformation occurred. Results are shown in Table 10.

**Table 10 Hygroscopicity test of the pharmaceutical co-crystals formed by the compound of formula (I)**

| Hygroscopicity at 25°C evaluated by DVS | | | | | | | | |
|---|---|---|---|---|---|---|---|---|
| Physical form | | | | | | | | |
| | Pharmaceutical co-crystal formed by the compound of formula (I) and the L-proline, anhydrous crystal form | | | | Pharmaceutical co-crystal formed by the compound of formula (I) and the nicotinamide, anhydrous crystal form | | | |
| Relative humidity at 25°C | Desorption (%) | Adsorption (%) | Desorption (%) | Adsorption (%) | Desorption (%) | Adsorption (%) | Desorption (%) | Adsorption (%) |
| 0% | 0.2 | 0.2 | 0.3 | 0.3 | 0.2 | 0.2 | 0.1 | 0.1 |
| 10% | 0.2 | 0.4 | 0.0 | 0.1 | 0.3 | 0.2 | 0.2 | 0.2 |
| 20% | 0.2 | 0.0 | 1.5 | 0.3 | 0.2 | 0.2 | 0.2 | 0.1 |
| 30% | 0.2 | 0.3 | 3.9 | 0.4 | 0.3 | 0.2 | 0.0 | 0.1 |
| 40% | 0.2 | 0.1 | 6.1 | 0.5 | 0.1 | 0.2 | 0.3 | 0.2 |
| 50% | // | 0.3 | 9.6 | // | // | 0.2 | 0.2 | // |
| 60% | // | 0.4 | 12.9 | // | // | 0.2 | 0.2 | // |
| 70% | // | 0.5 | 19.1 | // | // | 0.2 | 0.1 | // |
| 80% | // | 0.5 | 30.5 | // | // | 0.3 | 0.1 | // |
| 90% | // | 10.8 | 54.1 | // | // | 0.2 | 0.3 | // |
| 95% | // | 84.5 | 84.5 | // | // | 0.4 | 0.4 | // |
| Hygroscopicity | Slight hygroscopicity (40%-80% RH), and high hygroscopicity (80%-95% RH); water absorption of 0.4% from 40% to 80% RH, and water absorption of 84.0% from 80% to 95% RH | | | | Slight hygroscopicity, water absorption of 0.2% from 40% to 95% RH | | | |
| XRPD of samples after DVS testing | Free crystal form, L-proline, L-proline co-crystal, and unknown peaks | | | | Nicotinamide co-crystal | | | |
| Note: *"*//*":* not conducted | | | | | | | | |
| | no or almost no hygroscopicity: water absorption amount < 0.2% | | | | | | | |
| | slight hygroscopicity: water absorption amount ≥ 0.2% but < 2% | | | | | | | |
| | hygroscopicity: water absorption amount ≥ 2% but < 15% | | | | | | | |
| | high hygroscopicity: water absorption amount ≥ 15% | | | | | | | |
| | deliquescence: absorption of a sufficient amount of water to form a liquid | | | | | | | |
| | water absorption amount = water absorption amount at specific RH (80% to 95%) - water absorption amount at 40% RH | | | | | | | |
| Based on the description of hygroscopicity characteristics and the definition of hygroscopic weight gain in the "9103 Guidelines for the Hygroscopicity of Pharmaceuticals" in Part IV of the 2020 edition of the Chinese Pharmacopoeia. | | | | | | | | |

It can be known from Table 10 that the pharmaceutical co-crystal formed by the compound of formula (I) and the L-proline had slight hygroscopicity from 40% RH to 80% RH, with a hygroscopic weight gain of approximately 0.4%; and had high hygroscopicity from 80% RH to 95% RH, with a hygroscopic weight gain of approximately 84.0%. After the DVS testing, the co-crystal was partially dissociated into a free crystal form and L-proline. The pharmaceutical co-crystal formed by the compound of formula (I) and the nicotinamide had slight hygroscopicity, with a hygroscopic weight gain of approximately 0.2% from 40% RH to 95% RH at 25°C. After the DVS testing, no crystal form transformation occurred.

It should be understood that the above examples are all exemplary and are not intended to encompass all possible embodiments covered by the claims. Without departing from the scope of the present disclosure, various modifications and alterations may also be made based on the above examples. Similarly, various technical features of the above examples may also be arbitrarily combined to form other examples of the present disclosure that may not have been explicitly described. Therefore, the above examples merely express several embodiments of the present disclosure and do not limit the scope of protection of the present disclosure.

## Claims

1. A pharmaceutical co-crystal formed by a compound of formula (I) and a co-crystal former, wherein the co-crystal former is selected from nicotinamide, isonicotinamide, L-proline, and glycolic acid,

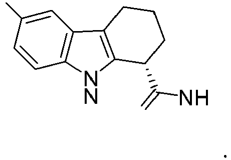

2. The pharmaceutical co-crystal according to claim 1, wherein a stoichiometric ratio of the compound of formula (I) to the co-crystal former is 1:0.5 to 1:3, preferably 1:0.5, 1:1, 1:2, or 1:3, and most preferably 1:1 or 1:2.

3. The pharmaceutical co-crystal according to claim 1, wherein the co-crystal former is the nicotinamide.

4. A pharmaceutical co-crystal formed by a compound of formula (I) and isonicotinamide, 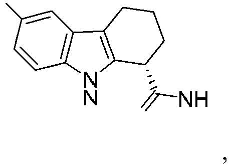 wherein an X-ray powder diffraction pattern expressed in terms of diffraction angle 2θ has characteristic peaks at 13.641, 14.019, 18.822, 21.196 and 26.566; preferably has characteristic peaks at 9.386, 11.847, 13.641, 14.019, 15.097, 18.822, 21.196, 23.762, 24.300, 26.566 and 35.936; preferably has characteristic peaks at 6.139, 9.386, 11.847, 13.641, 14.019, 15.097, 16.746, 17.556, 17.893, 18.822, 21.196, 23.762, 24.300, 26.354, 26.566, 33.296 and 35.936; and most preferably, the X-ray powder diffraction pattern expressed in terms of diffraction angle 2θ is as shown in FIG. 1.

5. A pharmaceutical co-crystal formed by a compound of formula (I) and glycolic acid, 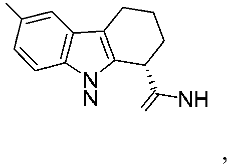 wherein an X-ray powder diffraction pattern expressed in terms of diffraction angle 2θ has characteristic peaks at 10.588, 17.645, 21.232, 21.527 and 23.125; preferably has characteristic peaks at 10.588, 12.575, 17.645, 18.231, 19.706, 21.232, 21.527, 23.125, 24.776, 25.300 and 27.608; preferably has characteristic peaks at 10.588, 12.575, 14.928, 17.645, 18.231, 18.444, 19.706, 21.232, 21.527, 23.125, 24.776, 25.300, 27.608, 28.937, 30.376 and 33.925; preferably has characteristic peaks at 10.588, 12.575, 14.928, 17.645, 18.231, 18.444, 19.706, 20.417, 21.232, 21.527, 23.125, 24.776, 25.300, 26.840, 27.608, 28.937, 30.116, 30.376, 32.070, 33.925, 35.159, 35.368 and 36.010; and most preferably, the X-ray powder diffraction pattern expressed in terms of diffraction angle 2θ is as shown in FIG. 4.

6. A pharmaceutical co-crystal formed by a compound of formula (I) and L-proline, 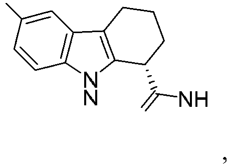 wherein an X-ray powder diffraction pattern expressed in terms of diffraction angle 2θ has characteristic peaks at 10.302, 14.229, 14.871, 19.762 and 20.643; preferably has characteristic peaks at 7.120, 10.302, 13.153, 14.229, 14.871, 19.762, 20.643, 23.211, 28.621 and 31.158; preferably has characteristic peaks at 6.585, 7.120, 10.302, 13.153, 14.229, 14.871, 19.762, 20.113, 20.643, 22.829, 23.211, 23.611, 26.465, 28.621, 31.158 and 37.176; preferably has characteristic peaks at 6.585, 7.120, 10.302, 13.153, 14.229, 14.871, 15.763, 16.443, 18.719, 19.762, 20.113, 20.643, 21.217, 21.383, 21.899, 22.829, 23.211, 23.611, 26.465, 28.621, 31.158 and 37.176; and most preferably, the X-ray powder diffraction pattern expressed in terms of diffraction angle 2θ is as shown in FIG. 7.

7. A pharmaceutical co-crystal formed by a compound of formula (I) and nicotinamide, 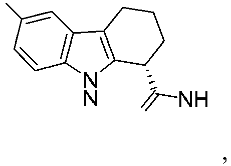 wherein an X-ray powder diffraction pattern expressed in terms of diffraction angle 2θ has characteristic peaks at 13.311, 17.017, 19.752, 23.638 and 26.573; preferably has characteristic peaks at 9.860, 13.311, 17.017, 19.752, 20.856, 23.638, 24.147, 26.573, 27.028 and 32.820; preferably has characteristic peaks at 9.860, 13.311, 17.017, 19.752, 20.856, 23.161, 23.638, 24.147, 24.780, 26.165, 26.573, 27.028, 30.766, 32.820 and 36.741; preferably has characteristic peaks at 9.860, 11.529, 13.311, 14.036, 16.007, 17.017, 17.503, 19.752, 20.856, 21.661, 23.161, 23.638, 24.147, 24.780, 26.165, 26.573, 27.028, 30.766, 32.284, 32.820 and 36.741; and most preferably, the X-ray powder diffraction pattern expressed in terms of diffraction angle 2θ is as shown in FIG. 10.

8. The pharmaceutical co-crystal according to any one of claims 1-7, wherein the 2θ angle has an error margin of ±0.20 degrees.

9. A method for preparing the pharmaceutical co-crystal formed by the compound of formula (I) and the isonicotinamide according to claim 4, comprising: mixing the compound of formula (I) with the isonicotinamide, adding solvent 1, stirring, and filtering; wherein
preferably, the solvent 1 is selected from one or more of alcohol, ester, nitrile, and water, preferably alcohol solvent, more preferably C₁₋₄ alcohol, more preferably one or more of methanol, ethanol, and isopropanol, and most preferably ethanol.

10. A method for preparing the pharmaceutical co-crystal formed by the compound of formula (I) and the glycolic acid according to claim 5, comprising: mixing the compound of formula (I) with the glycolic acid, adding solvent 2, stirring, and filtering; wherein
preferably, the solvent 2 is selected from one or more of alcohol, ester, nitrile, and water, preferably ester solvent, more preferably one or more of ethyl acetate, n-propyl acetate, isopropyl acetate, n-butyl acetate, and isobutyl acetate, and most preferably isopropyl acetate.

11. A method for preparing the pharmaceutical co-crystal formed by the compound of formula (I) and the L-proline according to claim 6, comprising: mixing the compound of formula (I) with the L-proline, adding solvent 3, stirring, and filtering; wherein
preferably, the solvent 3 is selected from one or more of alcohol, ester, nitrile, and water, preferably alcohol solvent, more preferably C₁₋₄ alcohol, more preferably one or more of methanol, ethanol, and isopropanol, and most preferably ethanol.

12. A method for preparing the pharmaceutical co-crystal formed by the compound of formula (I) and the nicotinamide according to claim 7, comprising: mixing the compound of formula (I) with the nicotinamide, adding solvent 4, stirring, and filtering; wherein
preferably, the solvent 4 is selected from one or more of alcohol, ester, nitrile, and water, preferably nitrile solvent, more preferably one or more of acetonitrile, trimethylacetonitrile, propionitrile, and valeronitrile, and most preferably acetonitrile.

13. A pharmaceutical composition prepared from the pharmaceutical co-crystal formed by the compound of formula (I) and the co-crystal former according to any one of claims 1-3, the pharmaceutical co-crystal formed by the compound of formula (I) and the isonicotinamide according to claim 4, the pharmaceutical co-crystal formed by the compound of formula (I) and the glycolic acid according to claim 5, the pharmaceutical co-crystal formed by the compound of formula (I) and the L-proline according to claim 6, or the pharmaceutical co-crystal formed by the compound of formula (I) and the nicotinamide according to claim 7.

14. A pharmaceutical composition, comprising the pharmaceutical co-crystal formed by the compound of formula (I) and the co-crystal former according to any one of claims 1-3, the pharmaceutical co-crystal formed by the compound of formula (I) and the isonicotinamide according to claim 4, the pharmaceutical co-crystal formed by the compound of formula (I) and the glycolic acid according to claim 5, the pharmaceutical co-crystal formed by the compound of formula (I) and the L-proline according to claim 6, or the pharmaceutical co-crystal formed by the compound of formula (I) and the nicotinamide according to claim 7, and optionally a pharmaceutically acceptable excipient.

15. A method for preparing a pharmaceutical composition, comprising the step of mixing the pharmaceutical co-crystal formed by the compound of formula (I) and the co-crystal former according to any one of claims 1-3, the pharmaceutical co-crystal formed by the compound of formula (I) and the isonicotinamide according to claim 4, the pharmaceutical co-crystal formed by the compound of formula (I) and the glycolic acid according to claim 5, the pharmaceutical co-crystal formed by the compound of formula (I) and the L-proline according to claim 6, or the pharmaceutical co-crystal formed by the compound of formula (I) and the nicotinamide according to claim 7, with a pharmaceutically acceptable excipient.

16. Use of the pharmaceutical co-crystal formed by the compound of formula (I) and the co-crystal former according to any one of claims 1-3, the pharmaceutical co-crystal formed by the compound of formula (I) and the isonicotinamide according to claim 4, the pharmaceutical co-crystal formed by the compound of formula (I) and the glycolic acid according to claim 5, the pharmaceutical co-crystal formed by the compound of formula (I) and the L-proline according to claim 6, the pharmaceutical co-crystal formed by the compound of formula (I) and the nicotinamide according to claim 7, the composition according to claims 13 or 14, or the composition prepared by the method according to claim 15 in preparation of a drug for treating and/or preventing a disease related to RNA m6A regulation.

17. The use according to claim 16, wherein the disease related to RNA m6A regulation is a disease or pain related to endocrine and metabolic diseases, neurological diseases, tumors, cardiovascular diseases, infections, immune system diseases, urogenital diseases, dermatological and musculoskeletal diseases, respiratory diseases, genetic diseases and malformations, digestive system diseases, stomatognathic diseases, and vascular and lymphatic system diseases, and is preferably a hand-foot syndrome, a hand-foot skin reaction, a cancer, or a dermatological disease.
